(19) Europäisches Patentamt
European Patent Office
Office européen des brevets

(11) **EP 2 688 905 B1**

(12) **FASCICULE DE BREVET EUROPEEN**

(45) Date de publication et mention de la délivrance du brevet:
**24.08.2016 Bulletin 2016/34**

(21) Numéro de dépôt: **12710514.6**

(22) Date de dépôt: **26.03.2012**

(51) Int Cl.:
*C07K 14/315* (2006.01)   *G01N 33/50* (2006.01)
*G01N 33/531* (2006.01)

(86) Numéro de dépôt international:
**PCT/EP2012/055333**

(87) Numéro de publication internationale:
**WO 2012/127060 (27.09.2012 Gazette 2012/39)**

(54) **PEPTIDES APTES À FORMER UN COMPLEXE COVALENT ET LEURS UTILISATIONS**

PEPTIDE ZUR HERSTELLUNG EINES KOVALENTEN KOMPLEXES UND IHRE VERWENDUNG

PEPTIDES CAPABLE OF FORMING A COVALENT COMPLEX, AND USES THEREOF

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **24.03.2011 FR 1152432**

(43) Date de publication de la demande:
**29.01.2014 Bulletin 2014/05**

(73) Titulaire: **Commissariat à l'Énergie Atomique et aux Énergies Alternatives 75015 Paris (FR)**

(72) Inventeurs:
• **VERNET, Thierry**
  **F-38000 Grenoble (FR)**
• **DI GUILMI, Anne-Marie**
  **F-38100 Grenoble (FR)**

(74) Mandataire: **Ahner, Philippe et al BREVALEX 95, rue d'Amsterdam 75378 Paris Cedex 8 (FR)**

(56) Documents cités:
**WO-A1-2011/098772**

• IZORE T ET AL: "Structural Basis of Host Cell Recognition by the Pilus Adhesin from Streptococcus pneumoniae", STRUCTURE, CURRENT BIOLOGY LTD., PHILADELPHIA, PA, US, vol. 18, no. 1, 13 janvier 2010 (2010-01-13), pages 106-115, XP026835220, ISSN: 0969-2126 [extrait le 2010-01-12]

• HU X ET AL: "Autocatalytic intramolecular isopeptide bond formation in Gram-positive bacterial pili: A QM/MM simulation", JOURNAL OF THE AMERICAN CHEMICAL SOCIETY, AMERICAN CHEMICAL SOCIETY, WASHINGTON, DC; US, vol. 133, no. 3, 26 janvier 2011 (2011-01-26), pages 478-485, XP002631440, ISSN: 0002-7863, DOI: 10.1021/JA107513T [extrait le 2010-12-13]

• ZAKERI BIJAN ET AL: "Spontaneous Intermolecular Amide Bond Formation between Side Chains for Irreversible Peptide Targeting", JOURNAL OF THE AMERICAN CHEMICAL SOCIETY, AMERICAN CHEMICAL SOCIETY, WASHINGTON, DC; US, vol. 132, no. 13, 7 avril 2010 (2010-04-07), pages 4526-4527, XP002631441, ISSN: 0002-7863, DOI: 10.1021/JA910795A [extrait le 2010-03-17]

• EL MORTAJI LAMYA ET AL: "Stability and Assembly of Pilus Subunits of Streptococcus pneumoniae", JOURNAL OF BIOLOGICAL CHEMISTRY, vol. 285, no. 16, avril 2010 (2010-04), pages 12405-12415, XP002667916, ISSN: 0021-9258

• BUDZIK JONATHAN M ET AL: "Amide bonds assemble pili on the surface of bacilli", PROCEEDINGS OF THE NATIONAL ACADEMY OF SCIENCES OF THE UNITED STATES OF AMERICA, vol. 105, no. 29, juillet 2008 (2008-07), pages 10215-10220, XP002667917, ISSN: 0027-8424

- KRISHNAN ET AL: "An IgG-like Domain in the Minor Pilin GBS52 of Streptococcus agalactiae Mediates Lung Epithelial Cell Adhesion", STRUCTURE, CURRENT BIOLOGY LTD., PHILADELPHIA, PA, US, vol. 15, no. 8, 14 août 2007 (2007-08-14), pages 893-903, XP022192470, ISSN: 0969-2126, DOI: 10.1016/J.STR.2007.06.015
- WIKOFF WILLIAM R ET AL: "Topologically linked protein rings in the bacteriophage HK97 capsid", SCIENCE (WASHINGTON D C), vol. 289, no. 5487, 22 septembre 2000 (2000-09-22), pages 2129-2133, XP002667918, ISSN: 0036-8075
- ZAKERI; HOWARTH: J. AM. CHEM. SOC., vol. 132, 2010, pages 4526-4527,
- ALEXANDRA S SOLOVYOVA ET AL: "Solution structure of the major (Spy0128) and minor (Spy0125 and Spy0130) pili subunits from Streptococcus pyogenes", EUROPEAN BIOPHYSICS JOURNAL ; WITH BIOPHYSICS LETTERS, SPRINGER, BERLIN, DE, vol. 39, no. 3, 17 March 2009 (2009-03-17) , pages 469-480, XP019778441, ISSN: 1432-1017

**Description**

**DOMAINE TECHNIQUE**

La présente invention concerne, de façon générale, le domaine des biotechnologies et notamment le domaine des outils utiles en biotechnologie.

Plus particulièrement, la présente invention propose deux nouveaux peptides, désignés ci-après Jo et In, capables de se lier l'un à l'autre de façon covalente et irréversible et dans des conditions douces, fournissant, de fait, des outils utiles non seulement dans le domaine de la détection, de la purification et de l'identification de composés d'intérêt mais aussi dans le domaine de l'ingénierie des protéines.

La présente invention concerne également l'hétérodimère correspondant à la fusion des peptides Jo et In ainsi qu'un élément tel qu'un support solide ou un composé marqué par au moins un des peptides Jo ou In et leurs utilisations.

**ÉTAT DE LA TECHNIQUE ANTÉRIEURE**

Le phénomène de reconnaissance spécifique entre molécules est à la base des systèmes biologiques et est utilisé dans de nombreuses applications en biotechnologie.

Cette reconnaissance entre macromolécules (protéines, acides nucléiques) ou entre macromolécules et molécules organiques de plus petite taille ou atomes se fait, dans la très grande majorité des cas, par l'intermédiaire de plusieurs liaisons chimiques non-covalentes.

Les procédés de purification, d'analyse et de détection de telles macromolécules mettent également en oeuvre de telles reconnaissances spécifiques. Par exemple, ces dernières impliquent le couple (strept)avidine/biotine, protéine A/immunoglobuline, protéine G/immunoglobuline, des couples anticorps/antigène ou anticorps/épitope comme le peptide poly-His et un anticorps spécifique de ce peptide ou le fragment C-terminal de la protéine Myc et l'anticorps monoclonal 9E10 ; des couples enzyme/substrat comme le couple glutathione S-transférase/glutathione ou des couples séquence nucléotidique/séquence nucléotidique complémentaire.

Toutefois, ces interactions non-covalentes impliquent des liaisons ioniques, des liaisons hydrogène, des liaisons hydrophobes et/ou des forces de Van der Waals. Ces interactions sont donc réversibles et non-permanentes. Ainsi, leur utilisation et leur mise en oeuvre est tributaire des conditions de stringence, de température et/ou de pH auxquelles les couples précités sont soumis.

La combinaison des phénomènes de reconnaissance spécifique de deux entités suivie par leur liaison spontanée et irréversible par la formation d'une liaison covalente serait un avantage considérable pour une grande gamme d'applications en biotechnologie et dans le domaine des matériaux innovants.

L'article de Zakeri et Howarth, 2010 (J. Am. Chem. Soc., vol. 132, pages 4526-4527) propose un moyen pour lier une étiquette peptidique de façon irréversible en mettant en oeuvre une liaison isopeptidique i.e. une liaison impliquant la chaîne latérale d'une lysine et la chaîne latérale d'une asparagine, non consécutives. Les travaux décrits dans cet article ont été menés sur la protéine Spy0128 de *Streptococcus pyogenes* présentant deux liaisons isopeptidiques : une entre la chaîne latérale de la Lys179 et la chaîne latérale de l'Asn303 et l'autre entre la chaîne latérale de la Lys36 et la chaîne latérale de l'Asn168.

La protéine Spy0128 a été divisée en deux fragments : le 1er correspond aux résidus 18-299 de la protéine Spy0128 et contenant la Lys179, alors que l'autre correspond aux résidus 293-308 et contient l'Asn303. Ces deux fragments produits de façon recombinante puis mélangés ensemble sont capables de se lier de façon covalente via la liaison peptidique entre Lys179 et Asn303. Cette liaison est spontanée, spécifique et intervient dans des conditions de pH compris entre 6 et 8 et de température comprise entre 4 et 37°C. La formation de cette liaison isopeptidique entre les deux fragments a également été montrée *in vivo.* L'autre liaison isopeptidique a été étudiée, de façon similaire, *in vitro,* en divisant la protéine Spy0128 en deux fragments chacun contenant un des acides aminés impliqués dans la liaison isopeptidique.

Il convient de souligner que l'article de Zakeri et Howarth, 2010 ne présente que des résultats obtenus avec des fragments qui correspondent ensemble à la protéine Spy0128 entière ou presque puisque seules de légères modifications des fragments décrits sont envisagées dans cet article.

L'article de Izoré et al., 2010 (Structure, vol. 18, pages 106-115) concerne la structure cristallographique à haute résolution de la protéine bactérienne RrgA (UniProt AC: Q97SC3). Cette adhésine est associée à la structure fibrillaire, qualifiée de pilus, présente à la surface de la bactérie pathogène humaine *Streptococcus pneumoniae.* Cette adhésine est décrite comme une adhésine à 4 domaines structuraux, désignés D1, D2, D3 et D4. Le domaine globulaire D2 se divise en deux régions séparées par le domaine D3, la 1ère région correspondant aux acides aminés 144-218, et la 2nde aux acides aminés 593-722, ces deux régions étant respectivement désignées région H1 et région H2. Dans la protéine RrgA, deux liaisons isopeptidiques intramoléculaires stabilisant la structure de cette dernière ont été identifiées. Une de ces liaisons se situe entre la Lys742 et l'Asn854 du domaine D4 et l'autre entre la Lys191 et l'Asn695 reliant de fait deux acides aminés appartenant aux deux régions du domaine D2 précédemment décrites.

Les inventeurs se sont donc fixés pour but de proposer des composés peptidiques permettant une reconnaissance spécifique, irréversible et spontanée et ce, dans des conditions douces et donc compatibles avec une utilisation *in vivo.*

## EXPOSÉ DE L'INVENTION

La présente invention permet de résoudre les problèmes techniques des éléments utilisés pour la reconnaissance spécifique de l'état de la technique et d'atteindre le but que se sont fixés les inventeurs.

En effet, les inventeurs ont poursuivi leurs travaux sur l'adhésine RrgA. Ils ont montré que la capacité d'association covalente entre la Lys191 et l'Asn695 est conservée lorsque deux régions particulières que sont la région H1 et la région H2, ci-après respectivement désignées domaine ou protéine Jo et domaine ou protéine In, sont isolées de leur protéine mère RrgA et produites sous forme recombinante chez la bactérie *E. coli* par les méthodes du génie génétique. Cette découverte n'est pas triviale car les deux domaines Jo et In sont très éloignés dans la séquence primaire d'acides aminés de RrgA. Il n'était donc pas évident pour l'homme du métier que d'autres éléments dans la séquence de RrgA ne soient pas nécessaires dans la formation de cette liaison isopeptidique, que cette dernière soit maintenue en absence des ¾ de la protéine RrgA et que ces deux domaines isolés puissent former cette liaison aussi bien *in vitro* qu'*in vivo.* De même, l'article de Zakeri et Howarth, 2010 ne suggère en aucun cas de travailler avec des domaines particuliers et restreints de la protéine Spy0128.

Plus particulièrement, les travaux des inventeurs ont montré que l'association entre les domaines Jo et In est très spécifique car contrairement à l'immense majorité des interactions moléculaires en biologie, celle-ci forme une liaison covalente irréversible. En conséquence, il est peu probable que des sous-produits résultant de l'association non désirée de Jo et In, avec d'autres substances naturelles apparaissent. Cette association peut avoir lieu *in vitro* ou *in vivo.* La liaison entre Jo et In est, de plus, covalente donc irréversible résultant en un complexe très stable via l'interaction entre ces domaines.

De plus, il a été mis en évidence que la réaction de liaison entre Jo et In est spontanée et a lieu dans des conditions douces sans nécessiter d'énergie et sans addition de réactifs coûteux ou complexes. De ce fait, cette association peut être mise en oeuvre *in vitro* ou dans un contexte cellulaire *in vivo.* Les inventeurs ont également montré que l'association covalente entre les domaines Jo et In induit une résistance du complexe à la protéolyse et à la dissociation par la chaleur, à la dissociation par l'action d'agents chaotropes, à la dissociation par modification de l'environnement acido-basique et à la dissociation par l'action de détergents.

Enfin, les peptides Jo et In associés entre eux cristallisent dans des conditions robustes en mettant en oeuvre des concentrations de l'ordre de 10 mg/ml et des solutions classiquement utilisées en cristallographie. Dans ces conditions, des cristaux présentant une bonne qualité de diffraction sont obtenus en 2-3 jours.

Compte tenu des propriétés remarquables ci-dessus exposées, le complexe JoIn constitue un outil aussi bien en biotechnologie, et notamment pour l'immunodétection et la purification par affinité mais également dans la fabrication de produits innovants tels que des polymères ou dans la cristallographie.

La présente invention est définie par les revendications attenantes. La description qui suit est soumise à cette limitation. Tous les aspects et formes de mise en oeuvre qui sont indiqués « selon la présente invention » mais qui ne sont pas couverts par les revendications sont présentés au titre de la divulgation et ne font pas partie de l'invention.

La présente invention concerne un peptide apte à former un complexe covalent consistant en :

- soit le peptide désigné peptide Jo, dont la séquence en acides aminés est la suivante :

    QYPQTGTYPDVQTPYQIIKVDGSEKNGQHKALNPNPYERVIPEGTLS KRIYQVNNLDDNQYGIELTVSGKTVYEQ (SEQ ID NO: 2 dans le listage de séquences en annexe), un dérivé ou un fragment de celui-ci :

- soit le peptide, désigné peptide In, dont la séquence en acides aminés est la suivante :

    IENGTITDPMGELIDLQLGTDGRFDPADYTLTANDGSRLENGQAVGG PQNDGGLLKNAKVLYDTTEKRIRVTGLYLGTDEKVTLTYNVRLNDEFVSNKFYDT NGRTTLHPKEVEQNTVRDFPIPKIRDVR (SEQ ID NO: 4 dans le listage de séquences en annexe), un dérivé ou un fragment de celui-ci.

Par « peptide apte à former un complexe covalent », on entend, dans le cadre de la présente invention, une entité de nature peptidique susceptible de former un complexe covalent et irréversible lorsque mise en présence du partenaire peptidique impliqué dans ce complexe. Les deux peptides objets de la présente invention constituent les deux partenaires à partir desquels un complexe peut être obtenu.

Le peptide Jo correspond à la séquence naturelle comprise entre les acides aminés 144-218 de] RrgA de *Streptococcus pneumoniae* et correspond donc au domaine défini comme la région H1 de RrgA.

Le peptide In correspond à la séquence naturelle comprise entre les acides aminés 593-722 de RrgA de *Streptococcus pneumoniae* et correspond donc au domaine défini comme région H2 de RrgA.

Dans la présente, les expressions « peptide Jo », « protéine Jo » et « Jo » sont équivalentes et utilisables de façon interchangeable. De même, les expressions « peptide In », « protéine In » et « In » sont équivalentes et utilisables de façon interchangeable.

Par « dérivé du peptide Jo » ou « dérivé du peptide In », on entend des peptides qui présentent au moins 40%, au moins 45%, au moins 50%, au moins 55%, au moins 60%, au moins 65%, au moins 70%, au moins 75%, au moins 80%, au moins 85%, au moins 90%, au moins 95% et/ou au moins 99% d'identité respectivement avec les séquences du peptide Jo et du peptide In données ci-dessus. Cette définition de dérivé couvre, par conséquent, les homologues des peptides Jo et In. Par « homologue du peptide Jo » ou « homologue du peptide In », on entend une forme différente ou équivalente du peptide Jo ou du peptide In, isolée d'une espèce différente de *Streptococcus pneumoniae.* Un exemple particulier d'un tel dérivé est un dérivé issu de la protéine PilA de *Streptococcus agalactiae.*

Par « pourcentage d'identité » entre deux séquences d'acides aminés (ou entre deux séquences nucléotidiques comme envisagé ci-après), on entend, dans le cadre de la présente invention, un pourcentage de résidus d'acides aminés (ou de nucléotides) identiques entre les deux séquences comparées, ce pourcentage étant obtenu après mise en oeuvre du meilleur alignement (alignement optimum) entre les deux séquences. L'homme du métier connaît différentes techniques permettant d'obtenir un tel pourcentage d'identité et impliquant des algorithmes d'homologie ou des programmes d'ordinateur tels que le programme BLAST.

Le pourcentage d'identité est statistique et les différences entre les deux séquences sont réparties aléatoirement le long de ces séquences. Les différences entre les deux séquences peuvent consister en différents types de modifications des séquences : des délétions, des substitutions ou des additions de résidus d'acides aminés (ou de nucléotides).

Ainsi, les modifications entre les peptides Jo et In et leurs dérivés peuvent consister en des substitutions, additions et/ou délétions d'un ou de plusieurs acides aminés. Les substitutions envisagées peuvent être des substitutions entre acides aminés équivalents i.e. des acides aminés présentant des homologies structurales ou ne modifiant pas substantiellement les propriétés du peptide Jo ou du peptide In. En variante, les substitutions envisagées peuvent être des substitutions par des acides aminés non équivalents i.e. des acides aminés ne présentant pas d'homologie structurale. Quel que soit le type de modifications mis en oeuvre, ces dernières n'affectent toutefois pas la capacité d'un dérivé du peptide Jo (ou d'un dérivé du peptide In) à covalemment se lier au moyen d'une liaison isopeptidique avec le peptide In, un fragment ou un dérivé de ce dernier (ou avec le peptide Jo, un fragment ou un dérivé de ce dernier).

Les dérivés des peptides Jo et In peuvent également présenter, par rapport aux séquences SEQ ID NO: 2 et SEQ ID NO: 4 données ci-dessus, au moins un acide aminé supplémentaire en partie C-terminale et/ou en partie N-terminale, une modification post-traductionnelle et/ou une modification chimique en particulier une glycosylation, une amidation, une acylation, une acétylation, une méthylation, ainsi que les peptides qui portent un groupement protecteur qui permet d'éviter leur dégradation. De façon avantageuse, les dérivés des protéines Jo et In peuvent présenter, par rapport aux séquences SEQ ID NO: 2 et SEQ ID NO: 4 données ci-dessus, 1, 2, 3, 4, 5, 6, 7, 8, 9 ou 10 acide(s) aminé(s) supplémentaire(s) en partie C-terminale et/ou 1, 2, 3, 4, 5, 6, 7, 8, 9 ou 10 acide(s) aminé(s) supplémentaire(s) en partie N-terminale. Un exemple particulier de tels dérivés correspond au dérivé du peptide Jo, dont la séquence en acides aminés est la suivante :

SDQYPQTGTYPDVQTPYQIIKVDGSEKNGQHKALNPNPYERVIPEGT       LSKRIYQVNNLDDNQYGIELTVSGKTVYEQKD (SEQ ID NO: 6 dans le listage de séquences en annexe) ;

En variante, les dérivés des protéines Jo et In peuvent présenter, par rapport aux séquences SEQ ID NO: 2 et SEQ ID NO: 4 données ci-dessus, 1, 2, 3, 4, 5, 6, 7, 8, 9 ou 10 acide(s) aminé(s) en plus en partie C-terminale et/ou 1, 2, 3, 4, 5, 6, 7, 8, 9 ou 10 acide(s) aminé(s) en moins en partie N-terminale.

En variante encore, les dérivés des protéines Jo et In peuvent présenter, par rapport aux séquences SEQ ID NO: 2 et SEQ ID NO: 4 données ci-dessus, 1, 2, 3, 4, 5, 6, 7, 8, 9 ou 10 acide(s) aminé(s) en moins en partie C-terminale et/ou 1, 2, 3, 4, 5, 6, 7, 8, 9 ou 10 acide(s) aminé(s) en plus en partie N-terminale.

Un exemple particulier de telles variantes est le dérivé du peptide In, dont la séquence en acides aminés est la suivante :

TEKKSIENGTITDPMGELIDLQLGTDGRFDPADYTLTANDGSRLENG QAVGGPQNDGGLLKNAKVLYDTTEKRIRVTGLYLGTDEKVTLTYNVRLNDEFVSN KFYDTNGRTTLHPKEVEQNTVRDFPIPKIRD (SEQ ID NO: 8 dans le listage de séquences en annexe).

Les dérivés des peptides Jo et In peuvent également être ceux dont un ou plusieurs acides aminés sont choisis dans le groupe constitué par des énantiomères, des diastéréoisomères, des acides aminés naturels de conformation D, des bêta acides aminés, des acides aminés alpha substitués, des acides aminés rares notamment l'hydroxyproline, l'hydroxylysine, l'allo-hydroxylysine, la 6-N-méthyllysine, la N-éthylglycine, la N-méthylglycine, la N-éthylasparagine, l'allo-isoleucine, la N-méthylisoleucine, la N-méthylvaline, la pyroglutamine, l'acide aminobutyrique et des acides aminés synthétiques notamment l'ornithine, la norleucine, la norvaline, la cyclohéxyl-alanine et les oméga-acides aminés. Les dérivés des peptides Jo et In couvrent selon l'invention également les rétropeptides et les rétroinversopeptides, de même que les peptides dont la chaîne latérale d'un ou plusieurs acides aminés est substituée par des groupements qui ne modifient pas la reconnaissance et la fusion d'un dérivé du peptide Jo avec un dérivé du peptide In.

Ainsi, quel que soit le type de dérivé envisagé, un dérivé du peptide Jo (ou un dérivé du peptide In) conserve sa capacité à covalemment se lier au moyen d'une liaison isopeptidique avec le peptide In, un fragment ou un dérivé de ce dernier (ou avec le peptide Jo, un fragment ou un dérivé de ce dernier).

Il est clair que ni le peptide Jo, ni le peptide In, ni un quelconque de leurs dérivés ou fragments ne couvre la protéine RrgA ou un de ses homologues en tant que tel(le).

Par « fragment du peptide Jo », on entend toute partie ou toute portion du peptide Jo ayant conservé sa capacité à covalemment se lier au moyen d'une liaison isopeptidique avec le peptide In. Un fragment du peptide Jo présente au moins un acide aminé en moins en partie C-terminale et/ou en partie N-terminale par rapport à la séquence SEQ ID NO: 2 du listage de séquences en annexe. Avantageusement, un fragment du peptide Jo correspond à la séquence en acides aminés comprise :

- entre les acides aminés 146 et 216 de la protéine RrgA (i.e. acides aminés 3 et 73 de SEQ ID NO: 2 du listage de séquences en annexe) ;
- entre les acides aminés 148 et 214 de la protéine RrgA (i.e. acides aminés 5 et 71 de SEQ ID NO: 2 du listage de séquences en annexe) ;
- entre les acides aminés 150 et 212 de la protéine RrgA (i.e. acides aminés 7 et 69 de SEQ ID NO: 2 du listage de séquences en annexe) ;
- entre les acides aminés 160 et 205 de la protéine RrgA (i.e. acides aminés 17 et 62 de SEQ ID NO: 2 du listage de séquences en annexe) ;
- entre les acides aminés 170 et 200 de la protéine RrgA (i.e. acides aminés 27 et 57 de SEQ ID NO: 2 du listage de séquences en annexe) ;
- entre les acides aminés 180 et 195 de la protéine RrgA (i.e. acides aminés 37 et 52 de SEQ ID NO: 2 du listage de séquences en annexe).

Par « fragment du peptide In », on entend toute partie ou toute portion du peptide In ayant conservé sa capacité à covalemment se lier au moyen d'une liaison isopeptidique avec le peptide Jo. Un fragment du peptide In présente au moins un acide aminé en moins en partie C-terminale et/ou en partie N-terminale par rapport à la séquence SEQ ID NO: 4 du listage de séquences en annexe. Avantageusement, un fragment du peptide In correspond à la séquence en acides aminés comprise :

- entre les acides aminés 595 et 720 de la protéine RrgA (i.e. entre les acides aminés 3 et 128 de SEQ ID NO: 4 du listage de séquences en annexe) ;
- entre les acides aminés 596 et 716 de la protéine RrgA (i.e. acides aminés 4 et 124 de SEQ ID NO: 4 du listage de séquences en annexe) ;
- entre les acides aminés 597 et 712 de la protéine RrgA (i.e. entre les acides aminés 5 et 120 de SEQ ID NO: 4 du listage de séquences en annexe) ;
- entre les acides aminés 598 et 708 de la protéine RrgA (i.e. acides aminés 6 et 116 de SEQ ID NO: 4 du listage de séquences en annexe) ;
- entre les acides aminés 599 et 704 de la protéine RrgA (i.e. entre les acides aminés 7 et 112 de SEQ ID NO: 4 du listage de séquences en annexe) ;
- entre les acides aminés 600 et 700 de la protéine RrgA (i.e. acides aminés 8 et 108 de SEQ ID NO: 4 du listage de séquences en annexe).

Ainsi, quel que soit le type de fragment envisagé, un fragment du peptide Jo (ou un fragment du peptide In) conserve sa capacité à covalemment se lier au moyen d'une liaison isopeptidique avec le peptide In, un fragment ou un dérivé de ce dernier (ou avec le peptide Jo, un fragment ou un dérivé de ce dernier).

Selon un mode de réalisation particulier, l'invention concerne :

- le peptide Jo de séquence SEQ ID NO: 6 ci-dessus, un dérivé ou un fragment de celui-ci apte à lier covalemment au moyen d'une liaison isopeptidique le peptide In (ou un fragment ou un dérivé de ce dernier) et/ou

- le peptide In de séquence SEQ ID NO: 8 ci-dessus, un dérivé ou un fragment de celui-ci apte à lier covalement au moyen d'une liaison isopeptidique le peptide Jo (ou un fragment ou un dérivé de ce dernier).

La présente invention concerne l'hétérodimère formé par les deux peptides de l'invention et tels que précédemment définis et liés l'un à l'autre par une liaison isopeptidique. En d'autres termes, cet hétérodimère comprend le peptide Jo de séquence SEQ ID NO: 2 ci-dessus, un dérivé ou un fragment de celle-ci et le peptide In de séquence SEQ ID NO: 4 ci-dessus, un dérivé ou un fragment de celle-ci, lesdits peptides Jo et In étant liés l'un à l'autre par une liaison isopeptidique.

Cet aspect de la présente invention couvre plus particulièrement un hétérodimère comprenant :

- le peptide Jo et le peptide In liés l'un à l'autre par une liaison isopeptidique ;
- un dérivé du peptide Jo et un dérivé du peptide In, liés l'un à l'autre par une liaison isopeptidique ;
- un fragment du peptide Jo et un fragment du peptide In, liés l'un à l'autre par une liaison isopeptidique.
- un dérivé du peptide Jo (ou In) et le peptide In (ou Jo) liés l'un à l'autre par une liaison isopeptidique ;
- un fragment du peptide Jo (ou In) et le peptide In (ou Jo) liés l'un à l'autre par une liaison isopeptidique ; et
- un dérivé du peptide Jo (ou In) et un fragment du peptide In (ou Jo) liés l'un à l'autre par une liaison isopeptidique.

Il est également clair que l'hétérodimère objet de la présente invention consiste en l'hétérodimère isolé et ne couvre donc pas l'hétérodimère naturellement inclus dans la protéine RrgA ou un de ses homologues.

La présente invention concerne également un polynucléotide isolé choisi parmi les différents polynucléotides ci-après :

i) un polynucléotide codant un peptide selon l'invention tel que précédemment défini ;
ii) un polynucléotide complémentaire du polynucléotide tel que défini au point (i) ;
iii) un polynucléotide d'au moins 18 nucléotides, capable de s'hybrider dans des conditions de forte stringence aux polynucléotides tels que définis aux points (i) et (ii).

Le polynucléotide selon l'invention ne correspond pas à une séquence nucléotidique dans son état naturel i.e. dans son environnement chromosomique naturel. Le polynucléotide ne correspond pas non plus au polynucléotide naturel codant la protéine RrgA de *Streptococcus pneumoniae* ou un de ses homologues. Au contraire, le polynucléotide selon l'invention a été isolé et éventuellement purifié, son environnement a, par conséquent, été modifié. Le polynucléotide selon l'invention peut également être obtenu par recombinaison génétique ou par synthèse chimique.

Les conditions de forte stringence correspondent à des conditions de température et de force ionique qui permettent de maintenir une hybridation entre deux séquences nucléotidiques complémentaires. L'homme du métier saura déterminer les conditions de forte stringence les mieux adaptées notamment en fonction de la taille des séquences nucléotidiques et ce, en se référant à l'enseignement de Sambrook et al. (Molecular cloning, 1989, Noland C. ed., New York : Cold Spring Harbor Laboratory Press).

Par « polynucléotide », on entend, dans le cadre de la présente invention, un acide nucléique, une séquence nucléique, une séquence d'acide nucléique, un oligonucléotide, une séquence polynucléotidique, une séquence nucléotidique, un ADN simple-brin, un ADN double-brin ou un ARN. Un polynucléotide selon la présente invention peut comprendre des nucléotides naturels et des nucléotides non naturels.

Le polynucléotide selon la présente invention comprend au moins une séquence nucléotidique présentant au moins 40%, au moins 45%, au moins 50%, au moins 55%, au moins 60%, au moins 65%, au moins 70%, au moins 75%, au moins 80%, au moins 85%, au moins 90%, au moins 95% et/ou au moins 99% d'identité avec une des séquences nucléotidiques ci-après ou une de leurs séquences complémentaires :

CAGTATCCACAAACAGGGACTTATCCAGATGTTCAAACACCTTATCA
GATTATTAAGGTAGATGGTTCGGAAAAAAACGGACAGCACAAGGCGTTGAATCCG
AATCCATATGAACGTGTGATTCCAGAAGGTACACTTTCAAAGAGAATTTATCAAG
TGAATAATTTGGATGATAACCAATATGGAATCGAATTGACGGTTAGTGGGAAAAC AGTGTATGAACAA (SEQ ID NO: 1 du listage de séquences en annexe) ou
ATTGAGAATGGTACGATTACAGATCCGATGGGTGAGTTAATTGATTT GCAATTGGGCACAGATGGAAGATT-
TGATCCAGCAGATTACACTTTAACTGCAAAC GATGGTAGTCGCTTGGAGAATGGACAAGCTGTAGGTGGTC-
CACAAAATGATGGTG    GTTTGTTAAAAAAATGCAAAAGTGCTCTATGATACGACTGAGAAAAGGATTCGTGT
AACAGGTCTGTACCTTGGAACGGATGAAAAAGTTACGTTGACCTACAATGTTCGT
TTGAATGATGAGTTTGTAAGCAATAAATTTTATGATACCAATGGTCGAACAACCT
TACATCCTAAGGAAGTAGAACAGAACACAGTGCGCGACTTCCCGATTCCTAAGAT TCGTGATCGTGAT (SEQ ID NO: 3 du listage de séquences en annexe).

Il est clair que le polynucléotide selon l'invention couvre également les séquences codant les fragments des peptides Jo et In précédemment envisagés, les séquences présentant au moins 40%, au moins 45%, au moins 50%, au moins 55%, au moins 60%, au moins 65%, au moins 70%, au moins 75%, au moins 80%, au moins 85%, au moins 90%, au moins 95% et/ou au moins 99% d'identité avec les séquences codant ces fragments et les séquences complémentaires de telles séquences. A partir des séquences de fragments envisagés et de la séquence nucléotidique codant les peptides Jo et In, il est facile pour l'homme du métier d'identifier la séquence nucléotidique codant un fragment particulier.

Il est également clair que le polynucléotide selon l'invention couvre également les séquences codant les dérivés des peptides Jo et In précédemment envisagés, les séquences présentant au moins 40%, au moins 45%, au moins 50%, au moins 55%, au moins 60%, au moins 65%, au moins 70%, au moins 75%, au moins 80%, au moins 85%, au moins 90%, au moins 95% et/ou au moins 99% d'identité avec les séquences codant ces dérivés et les séquences complémentaires de telles séquences. A partir des séquences de dérivés envisagés et de la séquence nucléotidique codant les peptides Jo et In, il est facile pour l'homme du métier d'identifier la séquence nucléotidique codant un dérivé particulier. Un exemple particulier de polynucléotides codant de tels dérivés correspond :

- soit, au polynucléotide codant le dérivé du peptide Jo, dont la séquence en acides aminés est la séquence SEQ ID NO: 6 dans le listage de séquences en annexe, ce polynucléotide présentant la séquence nucléotidique suivante :

      TCTGACCAGTATCCACAAACAGGGACTTATCCAGATGTTCAAACACC
      TTATCAGATTATTAAGGTAGATGGTTCGGAAAAAAACGGACAGCACAAGGCGTTG
      AATCCGAATCCATATGAACGTGTGATTCCAGAAGGTACACTTTCAAAGAGAATTT
      ATCAAGTGAATAATTTGGATGATAACCAATATGGAATCGAATTGACGGTTAGTGG     GAAAACAGTGTATGAACAAAAAGAT (SEQ ID NO: 5 du listage de séquences en annexe) et

- soit, au polynucléotide codant le dérivé du peptide In, dont la séquence en acides aminés est la séquence SEQ ID NO: 8 dans le listage de séquences en annexe, ce polynucléotide présentant la séquence nucléotidique suivante :

      ACTGAAAAGAAATCAATTGAGAATGGTACGATTACAGATCCGATGGG
      TGAGTTAATTGATTTGCAATTGGGCACAGATGGAAGATTTGATCCAGCAGATTAC
      ACTTTAACTGCAAACGATGGTAGTCGCTTGGAGAATGGACAAGCTGTAGGTGGTC
      CACAAAATGATGGTGGTTTGTTAAAAAATGCAAAAGTGCTCTATGATACGACTGA
      GAAAAGGATTCGTGTAACAGGTCTGTACCTTGGAACGGATGAAAAAGTTACGTTG
      ACCTACAATGTTCGTTTGAATGATGAGTTTGTAAGCAATAAATTTTATGATACCA
      ATGGTCGAACAACCTTACATCCTAAGGAAGTAGAACAGAACACAGTGCGCGACTT     CCCGATTCCTAA-
      GATTCGTGAT (SEQ ID NO: 7 du listage de séquences en annexe).

Avantageusement, le polynucléotide selon la présente invention est constitué d'une des séquences nucléotidiques ci-après ou une de leurs séquences complémentaires :

      CAGTATCCACAAACAGGGACTTATCCAGATGTTCAAACACCTTATCA
      GATTATTAAGGTAGATGGTTCGGAAAAAAACGGACAGCACAAGGCGTTGAATCCG
      AATCCATATGAACGTGTGATTCCAGAAGGTACACTTTCAAAGAGAATTTATCAAG
      TGAATAATTTGGATGATAACCAATATGGAATCGAATTGACGGTTAGTGGGAAAAC AGTGTATGAACAA (SEQ
      ID NO: 1 du listage de séquences en annexe),
      ATTGAGAATGGTACGATTACAGATCCGATGGGTGAGTTAATTGATTT  GCAATTGGGCACAGATGGAAGATT-
      TGATCCAGCAGATTACACTTTAACTGCAAAC GATGGTAGTCGCTTGGAGAATGGACAAGCTGTAGGTGGTC-
      CACAAAATGATGGTG      GTTTGTTAAAAAATGCAAAAGTGCTCTATGATACGACTGAGAAAAGGATTCGTGT
      AACAGGTCTGTACCTTGGAACGGATGAAAAAGTTACGTTGACCTACAATGTTCGT
      TTGAATGATGAGTTTGTAAGCAATAAATTTTATGATACCAATGGTCGAACAACCT
      TACATCCTAAGGAAGTAGAACAGAACACAGTGCGCGACTTCCCGATTCCTAAGAT TCGTGATCGTGAT (SEQ
      ID NO: 3 du listage de séquences en annexe),
      TCTGACCAGTATCCACAAACAGGGACTTATCCAGATGTTCAAACACC
      TTATCAGATTATTAAGGTAGATGGTTCGGAAAAAAACGGACAGCACAAGGCGTTG
      AATCCGAATCCATATGAACGTGTGATTCCAGAAGGTACACTTTCAAAGAGAATTT
      ATCAAGTGAATAATTTGGATGATAACCAATATGGAATCGAATTGACGGTTAGTGG     GAAAACAGTGTATGAA-
      CAAAAGAT (SEQ ID NO: 5 du listage de séquences en annexe) ou
      ACTGAAAAGAAATCAATTGAGAATGGTACGATTACAGATCCGATGGG    TGAGTTAATTGATTTGCAATTGGG-
      CACAGATGGAAGATTTGATCCAGCAGATTAC    ACTTTAACTGCAAACGATGGTAGTCGCTTGGAGAATGGA-

CAAGCTGTAGGTGGTC CACAAAATGATGGTGGTTTGTTAAAAAATGCAAAGTGCTCTATGATACGACTGA GAAAAGGATTCGTGTAACAGGTCTGTACCTTGGAACGGATGAAAAAGTTACGTTG ACCTACAATGTTCGTTTGAATGATGAGTTTGTAAGCAATAAATTTTATGATACCA ATGGTCGAACAACCTTACATCCTAAGGAAGTAGAACAGAACACAGTGCGCGACTT CCCGATTCCTAA-GATTCGTGAT (SEQ ID NO: 7 du listage de séquences en annexe).

La présente invention concerne également un vecteur de clonage et/ou d'expression contenant au moins un polynucléotide selon la présente invention. Un tel vecteur est notamment utile pour transformer un organisme hôte et exprimer, dans ce dernier, un peptide selon la présente invention.

Le vecteur selon la présente invention comprend en outre un (ou plusieurs) élément(s) qui permet(tent) l'expression du polynucléotide selon la présente invention et/ou la sécrétion du produit résultant de la traduction du polynucléotide selon la présente invention. Parmi ces éléments, on peut citer un promoteur constitutif ou inducible, un signal d'initiation de la transcription ou un signal de terminaison de la transcription, une séquence d'initiation de la traduction ou un signal de fin de traduction.

Avantageusement, le vecteur selon la présente invention comprend, liés entre eux de façon opérationnelle, un promoteur, un polynucléotide de l'invention et un élément terminateur. On entend par « liés entre eux de façon opérationnelle » selon l'invention, des éléments liés entre eux de façon à ce que le fonctionnement d'un des éléments soit affecté par celui d'un autre. A titre d'exemple, un promoteur est lié de façon opérationnelle à une séquence codante lorsqu'il est capable d'affecter l'expression de cette dernière. Les éléments régulateurs de la transcription, de la traduction et de la maturation des peptides que le vecteur peut comprendre sont connus de l'homme du métier et ce dernier est capable de les choisir en fonction de l'organisme hôte dans lequel l'expression ou le clonage doivent être réalisés.

Le vecteur selon la présente invention est avantageusement choisi parmi un plasmide, un cosmide, un bactériophage et un virus tel qu'un baculovirus. En particulier, le vecteur de l'invention est un vecteur à réplication autonome comportant des éléments permettant son maintien et sa réplication dans l'organisme hôte comme une origine de réplication. En outre, le vecteur peut comporter des éléments permettant sa sélection dans l'organisme hôte comme, par exemple, un gène de résistance à un antibiotique ou un gène de sélection qui assure la complémentation avec le gène respectif délété au niveau du génome de l'organisme hôte. De tels vecteurs de clonage et/ou d'expression sont bien connus de l'homme du métier et largement décrits dans la littérature.

L'invention concerne également un organisme hôte transformé par ou comprenant un polynucléotide selon la présente invention ou un vecteur selon la présente invention.

Par « organisme hôte », on entend tout organisme uni- ou pluricellulaire, inférieur ou supérieur, dans lequel un polynucléotide de l'invention est introduit pour la production d'un peptide selon la présente invention.

L'homme du métier connaît différentes méthodes pour introduire de façon efficace un polynucléotide dans un organisme hôte et ce, afin que, dans l'organisme hôte, le peptide codé par ledit polynucléotide soit produit. A titre d'exemple et de façon non exhaustive, cette méthode peut être une électroporation, une lipofection, une transformation biologique d'un végétal en utilisant *Agrobacterium tumefasciens,* un choc thermique ou un procédé chimique.

Avantageusement, l'organisme hôte est un micro-organisme tel qu'une levure, une bactérie ou un champignon. La transformation de tels micro-organismes permet de produire le peptide de l'invention à échelle semi-industrielle ou industrielle.

En variante, l'organisme hôte est une cellule animale telle qu'une cellule de mammifère, une cellule végétale, une cellule d'insecte, un animal à l'exception d'un humain, ou une plante.

De tels organismes hôtes peuvent être utilisés pour produire un peptide selon la présente invention. En effet, un procédé pour produire un peptide selon la présente invention comprend les étapes consistant à :

a) mettre en culture un organisme hôte selon la présente invention et notamment un organisme hôte unicellulaire dans un milieu de culture et dans des conditions appropriées ;
b) récupérer ledit peptide à partir du milieu de culture dudit organisme hôte cultivé ou à partir dudit organisme hôte cultivé.

La présente invention concerne un élément marqué par au moins un peptide selon l'invention et tel que précédemment défini.

Par « élément », on entend un composé ou un support solide auquel un peptide selon l'invention est attaché, lié, fixé et/ou associé ou avec lequel un peptide selon l'invention est complexé et/ou conjugué.

Dans une 1ère forme de mise en oeuvre, l'élément marqué selon la présente invention peut être un support solide à la surface duquel un (ou plusieurs) peptide(s) selon l'invention est(sont) immobilisé(s).

Dans une variante de l'invention, le support solide ou du moins la surface dudit support solide où le (ou les) peptide(s) selon l'invention est(sont) immobilisé (s) est notamment un support solide ou une surface inorganique. En effet, on peut envisager un support solide dont seule la surface est en un matériau inorganique particulier, le reste du support étant dans un autre matériau inorganique ou dans un matériau organique. Avantageusement, le support solide ou la surface dudit support solide est en un matériau inorganique choisi dans le groupe constitué par les verres, les quartz, les céramiques (par exemple, de type oxyde), les métaux (par exemple, aluminium, chrome, cuivre, zinc, argent, nickel, étain ou or), les métalloïdes (par exemple, silicium ou silicium oxydé) et leurs mélanges.

Dans une autre variante de l'invention, le support solide ou du moins la surface dudit support solide où le (ou les) peptide(s) selon l'invention est(sont) immobilisé(s) est en un matériau organique comme un polymère tel de l'agarose ou une résine incluant le nylon, le polyéthylène glycol, les polycarbonates, les polyfluoropolymères ou les composites. On peut également envisager un support solide dont seule la surface est en un matériau organique particulier, le reste du support étant dans un autre matériau organique ou dans un matériau inorganique.

Ledit support solide peut se présenter sous diverses formes de taille variable. A titre d'exemples et de façon non exhaustive, il peut se présenter sous forme de lames, de microplaques notamment de microplaques à 12, 24 ou 96 puits, de microplaquettes, de particules, de billes, de microbilles, de fibres, de feutres, de tubes tels que des tubes à hémolyse ou de microcanaux de type capillaire, des colonnes ou des microcolonnes telles que des colonnes SPIN™, de supports utilisés pour les biocapteurs ou les biopuces. Ces différents types de support peuvent avoir des tailles variant de quelques centaines de micromètres à plusieurs centimètres.

Avantageusement, le support solide présente une surface portant des groupements fonctionnels grâce auxquels le (ou les) peptide(s) selon l'invention est(sont) capable(s) de s'immobiliser. De façon particulière, ces groupements fonctionnels sont choisis parmi les groupements carboxyliques, des entités radicalaires, les fonctions alcools, aminés, amides, époxy ou thiols. Cette fonctionnalisation peut être intrinsèque à la nature du matériau en surface du support solide mis en oeuvre. De façon alternative, cette fonctionnalisation peut être obtenue par nettoyage de ladite surface par l'intermédiaire d'au moins un solvant, détergent, rayonnement ou plasma d'oxygène ou tout autre procédé permettant la formation de groupements fonctionnels tels que précédemment définis.

Dans une 1ere variante du support fonctionnalisé de la présente invention, le peptide selon l'invention peut être immobilisé de façon directe à la surface du support solide fonctionnalisé ou non. Un support solide « coaté » avec un peptide Jo ou In selon l'invention est un exemple d'immobilisation directe. Dans cette variante, le peptide selon l'invention est avantageusement lié à la surface du support solide fonctionnalisé ou non au moyen d'une liaison covalente. Ainsi, cette liaison covalente lie un atome de la surface du support solide, fonctionnalisé ou non, à un atome du peptide selon l'invention.

Dans une 2nde variante du support fonctionnalisé de la présente invention, le peptide selon l'invention peut être immobilisé, de façon indirecte, à la surface du support solide, fonctionnalisé ou non. Cette immobilisation indirecte fait intervenir un bras espaceur (ou agent de jonction) lié, d'une part, au support solide et, d'autre part, au peptide selon l'invention. L'homme du métier connaît différents exemples de tels bras espaceurs. De façon non exhaustive, peuvent être cités, en tant que bras espaceurs susceptibles d'être mis en oeuvre dans le cadre de la présente invention, le 1,6 diaminohexane, l'acide 6-aminohexanoïque, un groupe succinimide, un époxyde, l'acide UDP-glucuronique, des chaînes alkyles linéaires ou ramifiées de 1 à 20 atomes de carbone, du pyrrole, des silanes, du polyéthylène glycol, du glutaraldéhyde, etc...

Que l'immobilisation sur le support solide soit directe ou indirecte, le peptide selon l'invention peut être immobilisé via son extrémité N-terminale, via son extrémité C-terminale ou via la chaîne latérale d'un des acides aminés le constituant. Dans tous les cas, la seule contrainte est que la liaison avec son partenaire (i.e. peptide Jo, peptide In, leurs dérivés ou leurs fragments) ne soit pas affectée.

Les liaisons mises en oeuvre au cours d'une immobilisation directe ou indirecte peuvent être toutes liaisons connues de l'homme du métier et notamment des liaisons covalentes, des liaisons ioniques, des liaisons hydrogène, une adsorption, etc...

Dans une 2nde forme de mise en oeuvre, l'élément marqué selon la présente invention peut être un composé conjugué avec au moins un peptide selon l'invention et tel que précédemment défini.

Le composé tel que mis en oeuvre dans la présente invention est notamment une molécule naturelle ou synthétique telle qu'une molécule biologique ou biologiquement active, un monomère, un composé facilement détectable ou un composé cytotoxique.

Par « molécules biologiques ou biologiquement actives » sont des molécules naturelles ou synthétiques, avantageusement choisies parmi les épitopes ; les antigènes ; les peptides ; les oligopeptides ; les protéines telles qu'une enzyme ; les anticorps et fragments d'anticorps ; les récepteurs cellulaires ou membranaires ; des polysaccharides ; des cellules ou parties cellulaires telles que des organites ou des membranes cellulaires et les molécules nucléiques.

Parmi les molécules biologiques ou biologiquement actives, un oligopeptide avantageusement utilisé dans le cadre de la présente invention résulte de la fusion d'au moins deux peptides selon l'invention, identiques ou différents. Un tel oligopeptide répond à la formule (I) ci-après :

$$[(Jo)_m-(In)_n]_r \qquad (I)$$

dans laquelle :

m représente un nombre entier compris entre 0 et 50, n représente un nombre entier compris entre 0 et 50 et r représente un nombre entier compris entre 1 et 50,

lorsque m est égal à 0, n représente un nombre entier compris entre 1 et 50 ;

lorsque n est égal à 0, m représente un nombre entier compris entre 1 et 50 ;

lorsque r représente un nombre entier supérieur ou égal à 2, pour chaque répétition i avec i représentant un nombre entier compris entre 1 et (r-1), mi représentant la valeur m dans la répétition i est identique ou différent au m(i+1) représentant la valeur m dans la répétition i+1 et ni représentant la valeur n dans la répétition i est identique ou différent au n(i+1) représentant la valeur n dans la répétition i+1.

Par « molécules nucléiques », on entend un ADN simple ou double brin, un ARN, un ARNi, un aptamère, un PNA (pour « Peptide Nucleic Acid ») ou un LNA (pour « Locked Nucleic Acid »).

Par « composé facilement détectable », on entend, dans le cadre de la présente invention, un composé qui peut être détecté par mise en oeuvre d'une technique de détection appropriée avantageusement non invasive telle que la microscopie, la scintigraphie et l'imagerie par résonance magnétique (IRM). Un élément selon l'invention comprenant un tel composé facilement détectable est particulièrement adapté au domaine de l'imagerie et du diagnostic. Il permet notamment d'identifier et localiser des sites au niveau desquels le peptide Jo (ou le peptide In) est présent, ledit élément consistant en un composé facilement détectable conjugué avec le peptide In (ou le peptide Jo). Dans cette application, la présence du peptide Jo (ou du peptide In) au niveau d'un site particulier peut être le résultat de l'expression au niveau de ce site d'une protéine de fusion correspondant à une protéine particulière comme un récepteur membranaire, fusionnée directement ou indirectement au peptide Jo (ou au peptide In).

A titre d'exemples particuliers de composés facilement détectables, on peut citer :

- une enzyme ou une molécule capable de générer un signal détectable et éventuellement quantifiable dans des conditions particulières comme lors de la mise en présence d'un substrat adapté, comme la biotine, la digoxygénine, la 5-bromodéoxyuridine, une phosphatase alcaline, une peroxydase, une acétylcholine estérase (AChE), une glucose amylase et une lysozyme ; ou

- un marqueur fluorescent, chimiofluorescent ou bioluminescent tels que la fluorescéine et ses dérivés, la rhodamine et ses dérivés, la GFP (pour « Green Fluorescent Protein ») et ses dérivés, et l'umbelliférone ; le luminol ; la luciférase et la luciférine ;

- un marqueur radioactif, pouvant être introduit dans la séquence peptidique du peptide selon l'invention ou pouvant être porté par un composé distinct du peptide selon l'invention et conjugué à ce dernier. Dans le cas de la 1ère alternative (i.e. marqueur radioactif introduit dans le peptide selon l'invention), cette introduction peut avoir lieu lors de la synthèse du peptide selon l'invention en utilisant un ou plusieurs acides aminés marqués. En variante, cette introduction peut avoir lieu suite à cette synthèse en fixant le marqueur radioactif sur des résidus de la séquence peptidique du peptide selon l'invention synthétisé. Par exemple, l'yttrium-90 peut être fixé *via* un résidu lysine. Dans la 2[nde] alternative, le marqueur radioactif peut être indirectement fixé au peptide selon l'invention par des moyens connus. Par exemple, de l'EDTA ou un autre agent chélateur peut être fixé au peptide selon l'invention et utilisé pour fixer l'indium-111.

Par « composé cytotoxique », on entend, dans le cadre de la présente invention, un composé directement ou indirectement toxique. Par « directement cytotoxique », on entend un composé qui est en lui-même cytotoxique. Par « indirectement cytotoxique », on entend un composé qui, bien que non cytotoxique en lui-même, peut induire une cytotoxicité, par exemple par son action sur une autre molécule ou par une action supplémentaire sur lui.

A titre d'exemples particuliers de composés cytotoxiques, on peut citer :

- un agent chimiothérapeutique cytotoxique dont l'activité peut éventuellement être augmentée sous irradiation, tel que les agents alkylants comme la méchloréthamine ou le chlorambucile ; le méthotrexate ; la 5-fluoro-uracile ; la vinblastine ; la gemcitabine ; la fludarabine ; la nicotinamide ; la doxorubicine ; la mitomycine ; la L-asparaginase ; le cisplatine ; le taxol et ses analogues/dérivés ;

- un groupement (poly)peptidique cytotoxique tel que la ricine, l'abrine, l'exotoxine de Pseudomonas, le TNFα et l'interleukine 2 ;

- un agent chimiothérapeutique indirectement cytotoxique, également appelé pro-drogue, qui n'est pas ou peu cytotoxique en tant que tel mais est apte à donner, notamment suite à une réaction enzymatique ou à une irradiation, une substance cytotoxique (ou drogue) notamment telle que précédemment définie. Des exemples de pro-drogues sont notamment la méthotrexate-alanine ; la mitomycine phosphate, la 5-fluorocytosine ; la photofrine et la capécitabine ;

- un groupement (poly)peptidique indirectement cytotoxique qui présente une activité enzymatique et peut convertir une pro-drogue relativement non toxique notamment telle que précédemment définie en une substance cytotoxique. Des exemples de pro-drogues sont notamment une peptidase telle qu'une carboxypeptidase, une aminopeptidase ou une endopeptidase ; une phosphatase ; une sulfatase ; une amidase ; une kinase ; une glycosidase ; une désaminase ; une réductase ; et une oxydase ;

- une molécule d'acide nucléique qui est directement ou indirectement cytotoxique telle qu'un oligonucléotide anti-sens ou un aptamère.

Dans cette 2nde forme de mise en oeuvre, l'élément marqué selon la présente invention peut être un composé conjugué par un seul peptide tel que précédemment défini. La conjugaison avec le peptide peut être directe ou indirecte en mettant en oeuvre un bras espaceur tel que précédemment défini pour l'immobilisation d'un peptide de l'invention sur un support solide.

En variante, l'élément marqué selon la présente invention peut être un composé conjugué par deux peptides, identiques ou différents, tels que précédemment définis. Chacun des peptides peut être, indépendamment l'un de l'autre, directement ou indirectement lié au composé.

Dans cette variante, on peut donc avoir les cas de figures suivants :

- un composé conjugué directement avec le peptide Jo, un dérivé ou un fragment de celui-ci et directement avec le peptide In, un dérivé ou un fragment de celui-ci ;
- un composé conjugué indirectement avec le peptide Jo, un dérivé ou un fragment de celui-ci et directement avec le peptide In, un dérivé ou un fragment de celui-ci ;
- un composé conjugué directement avec le peptide Jo, un dérivé ou un fragment de celui-ci et indirectement avec le peptide In, un dérivé ou un fragment de celui-ci ;
- un composé conjugué indirectement avec le peptide Jo, un dérivé ou un fragment de celui-ci et indirectement avec le peptide In, un dérivé ou un fragment de celui-ci ;
- un composé conjugué directement avec un 1er peptide Jo, un dérivé ou un fragment de celui-ci et directement avec un 2nd peptide Jo, un dérivé ou un fragment de celui-ci ;
- un composé conjugué directement avec un 1er peptide Jo, un dérivé ou un fragment de celui-ci et indirectement avec un 2nd peptide Jo, un dérivé ou un fragment de celui-ci ;
- un composé conjugué indirectement avec un 1er peptide Jo, un dérivé ou un fragment de celui-ci et indirectement avec un 2nd peptide Jo, un dérivé ou un fragment de celui-ci ;
- un composé conjugué directement avec un 1er peptide In, un dérivé ou un fragment de celui-ci et directement avec un 2nd peptide In, un dérivé ou un fragment de celui-ci ;
- un composé conjugué directement avec un 1er peptide In, un dérivé ou un fragment de celui-ci et indirectement avec un 2nd peptide In, un dérivé ou un fragment de celui-ci ;
- un composé conjugué indirectement avec un 1er peptide In, un dérivé ou un fragment de celui-ci et indirectement avec un 2nd peptide In, un dérivé ou un fragment de celui-ci.

Que la conjugaison avec le composé soit directe ou indirecte, le (ou les) peptide(s) selon l'invention peu(ven)t être conjugué(s) via son(leurs) extrémité(s) N-terminale(s), via son(leurs) extrémité(s) C-terminale(s) ou via la chaîne latérale d'un des acides aminés le (s) constituant. Dans tous les cas, la seule contrainte est que la liaison avec son partenaire (i.e. peptide Jo, peptide In, leurs fragments ou leurs dérivés) ne soit pas affectée.

Les liaisons mises en oeuvre au cours d'une conjugaison directe ou indirecte peuvent être toutes liaisons connues de l'homme du métier et notamment des liaisons covalentes, des liaisons ioniques, des liaisons hydrogène, une adsorption, etc...

Dans une forme de mise en oeuvre particulière, un peptide selon l'invention peut être conjugué via son extrémité N-terminale, directement ou indirectement, à un 1er composé tel que précédemment défini et via son extrémité C-terminale, directement ou indirectement, à un 2nd composé tel que précédemment défini, identique ou différent au 1er composé.

L'homme du métier connaît différentes techniques permettant de conjuguer de tels composés à un (ou plusieurs) peptide(s) selon la présente invention une fois ce dernier obtenu ou produit ou préalablement à son obtention.

Ces techniques permettent un couplage covalent entre un peptide selon l'invention et un composé en mettant à profit des groupes chimiques particuliers portés par le peptide selon l'invention et par le composé. Parmi ces groupes chimiques particuliers, on peut citer un groupe thiol, un groupe ester, un groupe amino, un groupe acide et tout élément chimique susceptible d'être mis en oeuvre dans la « click-chemistry ».

En variante et notamment lorsque le composé est de nature peptidique, cette conjugaison peut consister à produire le composé conjugué selon l'invention sous forme d'un composé de fusion par les techniques de recombinaison génétique, dans lesquelles un polynucléotide comprend des régions respectives codant le peptide selon la présente invention et le composé, adjacentes l'une à l'autre, juxtaposées ou séparées par une région codant un bras espaceur tel qu'un lieur peptidique qui ne détruit pas les propriétés souhaitées du composé de fusion final.

De même, lorsque le composé selon l'invention est conjugué par deux peptides selon l'invention et que ce composé est de nature peptidique, on peut également envisager une production au moyen d'un polynucléotide comprenant une région codant le 1er peptide selon l'invention puis une région codant le composé et enfin une région codant le 2nd peptide selon l'invention, chacune des régions pouvant être juxtaposée avec la région qui la suit ou séparée de cette dernière par une région codant un bras espaceur du type lieur peptidique.

De même encore, lorsque le peptide selon l'invention est conjugué par deux composés et que ces composés sont de nature peptidique, on peut également envisager une production au moyen d'un polynucléotide comprenant une région codant le 1er composé puis une région codant le peptide selon l'invention et enfin une région codant le 2nd composé, chacune des régions pouvant être juxtaposée avec la région qui la suit ou séparée de cette dernière par une région codant un bras espaceur du type lieur peptidique.

Si deux bras espaceurs sont mis en oeuvre sur un même conjugué, ces derniers peuvent être identiques ou différents.

Qu'elle que soit la technique utilisée pour conjuguer un peptide selon la présente invention avec un composé, la seule contrainte à respecter dans le cadre de cette conjugaison est que le peptide conjugué i.e. le peptide Jo (ou le peptide In) conjugué conserve sa spécificité de liaison avec l'autre peptide selon l'invention i.e. le peptide In (ou le peptide Jo).

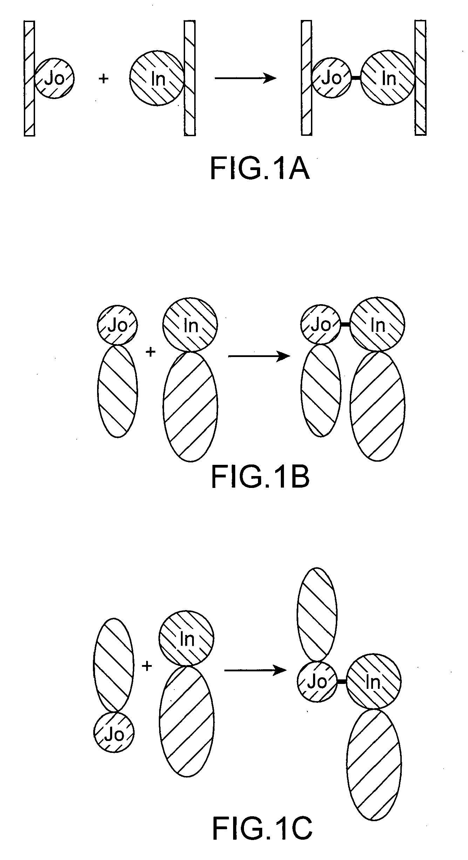

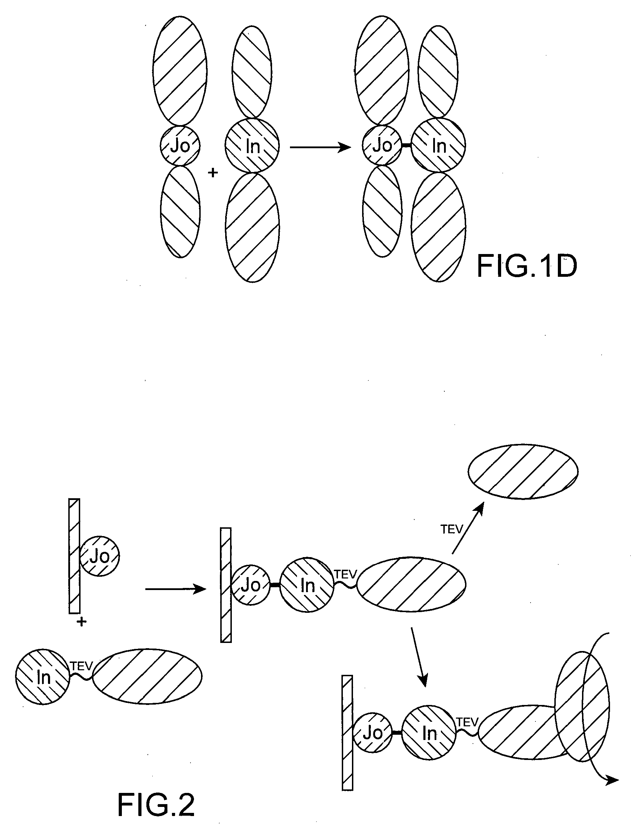

Les deux peptides selon la présente invention via leur capacité à former un complexe covalent et irréversible jouent le rôle de colle moléculaire, utile pour assembler deux surfaces (Figure 1A), deux supports solides, deux composés (Figures 1B, 1C et 1D), une surface et un support solide, une surface et un composé ou un support solide et un composé, ledit support solide, ladite surface et ledit composé étant tel(le) que précédemment défini(e) i.e. un support solide, une surface et un composé fonctionnalisé(e), directement ou indirectement, avec au moins un peptide selon l'invention.

La présente invention concerne l'utilisation d'un support solide selon la présente invention et d'un composé conjugué à au moins un peptide selon l'invention pour purifier ledit composé ou pour préparer une biopuce ou un biocapteur.

En effet, le composé peut être un composé d'intérêt pouvant être purifié en utilisant un support solide selon la présente invention. Dans ce cas, le composé d'intérêt est avantageusement conjugué à un seul peptide selon l'invention. Ce procédé pour purifier un composé d'intérêt comprend les étapes consistant en :

- conjuguer ledit composé d'intérêt avec un peptide selon la présente invention (i.e. avec soit le peptide Jo, un dérivé ou un fragment de celui-ci, soit le peptide In, un dérivé ou un fragment de celui-ci) ;
- mettre le composé d'intérêt ainsi conjugué avec un support solide sur lequel est immobilisé au moins un autre peptide selon la présente invention (i.e. soit le peptide In, un dérivé ou un fragment de celui-ci, si le composé est conjugué avec le peptide Jo, un dérivé ou un fragment de celui-ci ; soit le peptide Jo, un dérivé ou un fragment de celui-ci, si le composé est conjugué avec le peptide In, un dérivé ou un fragment de celui-ci). Dans ce procédé de purification, le support solide mis en oeuvre est avantageusement une colonne de purification, une résine ou une matrice habituellement utilisée en chromatographie d'affinité.

Pour une telle application, il peut être avantageux d'utiliser un composé d'intérêt indirectement conjugué à un peptide selon l'invention. En effet, un bras espaceur entre le composé d'intérêt et le peptide selon l'invention et notamment un bras espaceur peptidique susceptible d'être enzymatiquement clivé permet de récupérer le composé d'intérêt, suite à l'action de l'enzyme clivante telle qu'une protéase.

Ainsi, la présente invention concerne un procédé pour purifier un composé, comprenant les étapes consistant à :

- conjuguer indirectement ledit composé avec un des deux peptides selon la présente invention (i.e. avec soit le peptide Jo, un dérivé ou un fragment de celui-ci, soit le peptide In, un dérivé ou un fragment de celui-ci), un bras espaceur clivable séparant ledit composé et ledit peptide ;
- préparer un support solide à la surface duquel l'autre des deux peptides selon la présente invention (i.e. soit le peptide In, un dérivé ou un fragment de celui-ci, si le composé est conjugué avec le peptide Jo, un dérivé ou un fragment de celui-ci ; soit le peptide Jo, un dérivé ou un fragment de celui-ci, si le composé est conjugué avec le peptide In, un dérivé ou un fragment de celui-ci) est immobilisé ;
- mettre le composé ainsi conjugué en présence du support ainsi préparé, moyennant quoi le composé conjugué est immobilisé sur le support solide, cette immobilisation impliquant une liaison isopeptidique ;

- soumettre le composé ainsi immobilisé à des conditions permettant son clivage ; et
- récupérer le composé clivé purifié.

La présente invention concerne également un kit de purification comprenant :

- un support solide à la surface duquel un des deux peptides selon la présente invention (i.e. soit le peptide In, un dérivé ou un fragment de celui-ci ; soit le peptide Jo, un dérivé ou un fragment de celui-ci) est immobilisé ;
- un élément choisi parmi un peptide correspondant à l'autre peptide selon l'invention ; un polynucléotide tel que précédemment défini codant ledit peptide ou un vecteur d'expression et/ou de clonage tel que précédemment défini contenant ledit polynucléotide.

Il est clair que l'autre élément du kit de purification selon l'invention est à associer, à conjuguer ou à fusionner avec le composé à purifier ou avec une séquence nucléotidique codant un tel composé.

Par « l'autre peptide », on entend soit le peptide In, un dérivé ou un fragment de celui-ci, si le peptide Jo, un dérivé ou un fragment de celui-ci est immobilisé sur le support ; soit le peptide Jo, un dérivé ou un fragment de celui-ci, si le peptide In, un dérivé ou un fragment de celui-ci est immobilisé sur le support.

De même, la présente invention peut être utilisée pour la préparation de biopuces ou de biocapteurs. Cette préparation peut présenter différents modes de réalisation :

- préparer un support solide sur lequel est immobilisé au moins un peptide membre de l'hétérodimère selon la présente invention (i.e. soit le peptide Jo, un dérivé ou un fragment de celui-ci ; soit le peptide In, un dérivé ou un fragment de celui-ci) puis
- déposer un composé conjugué à au moins un peptide, autre membre de l'hétérodimère selon la présente invention (i.e. soit le peptide In, un dérivé ou un fragment de celui-ci, si, sur le support solide, le peptide immobilisé est le peptide Jo, un dérivé ou un fragment de celui-ci ; soit le peptide Jo, un dérivé ou un fragment de celui-ci, si, sur le support solide, le peptide immobilisé est le peptide In, un dérivé ou un fragment de celui-ci) sur la surface totale du support ; ou
- déposer ce composé conjugué grâce à un système de micro ou nano-fluidique de façon directe ou séquentielle sous la forme de gouttes ; ou
- tremper le support solide sur lequel est immobilisé au moins un peptide selon la présente invention dans la solution contenant ce composé conjugué.

Dans le cadre de la préparation de cette biopuce ou de ce biocapteur, le composé conjugué au peptide selon l'invention peut être tout composé habituellement utilisé dans ce type d'application. Plus particulièrement, ce composé est une molécule biologique ou biologiquement active telle que précédemment définie.

De plus, ce composé peut être conjugué par un ou deux peptides selon la présente invention. En effet, on peut envisager l'obtention d'une biopuce ou d'un biocapteur présentant, à leur surface, deux composés liés à l'un à l'autre. Un exemple particulier d'une telle variante consiste à utiliser un support solide sur lequel est immobilisée le peptide Jo, un dérivé ou un fragment de celui-ci et à le mettre en contact avec une solution contenant un composé A marqué par le peptide Jo, un dérivé ou un fragment de celui-ci et par le peptide In, un dérivé ou un fragment de celui-ci et un composé B marqué par le peptide In, un dérivé ou un fragment de celle-ci, ladite solution contenant, du fait des propriétés remarquables d'interaction entre Jo et In, un composé résultant de la liaison du composé A avec le composé B. En d'autres termes, le complexe AB est formé préalablement à la mise en contact avec le support solide. En variante, on peut envisager d'appliquer sur le support solide une 1$^{ère}$ solution contenant le composé A, puis une 2$^{nde}$ solution contenant le composé B.

La présente invention concerne également la biopuce ou le biocapteur susceptible d'être préparé(e) selon le procédé de préparation de la présente invention i.e. en utilisant un support solide et un composé conjugué selon la présente invention. En effet, cette biopuce et ce biocapteur se distinguent des biopuces et biocapteurs de l'état de la technique par la présence entre le support solide et le composé immobilisé à sa surface de l'hétérodimère Jo/In.

L'utilisation de la présente invention pour purifier des composés d'intérêt ou pour préparer des biopuces et des biocapteurs présente de nombreux avantages. Elle ne nécessite pas de modification chimique du composé d'intérêt, elle permet le contrôle de l'orientation du composé d'intérêt à la surface du support solide et permet que le composé d'intérêt soit lié de façon stable à cette surface.

En outre, lorsque le composé d'intérêt est de nature peptidique et que sa conjugaison à au moins un peptide selon l'invention est obtenue au moyen d'une protéine de fusion, la préparation de biopuces ou de biocapteurs peut être réalisée sur des lysats non purifiés contenant le composé d'intérêt de nature peptidique.

Dans cette schématisation de la Figure 2, le peptide Jo (Jo) est liée par réaction chimique à la surface d'un support solide et le composé selon l'invention est une protéine d'intérêt conjuguée au peptide In (In) mais séparé de cette dernière par un bras espaceur présentant le site de coupure spécifique de la cystéine protéase TEV (TEV). Une fois le composé selon l'invention mis au contact du support solide selon l'invention, le complexe obtenu peut être soumis à l'action de la cystéine protéase TEV, libérant ainsi la protéine d'intérêt (cas de la purification). En variante, le complexe constitue un biocapteur ou une biopuce pour étudier des interactions impliquant la protéine d'intérêt.

La présente invention concerne aussi un composé selon la présente invention i.e. un composé conjugué à un peptide selon la présente invention pour utilisation en médecine ou pour le diagnostic.

La présente invention comprend également un kit de diagnostic ou de traitement comprenant deux composés selon la présente invention. Plus particulièrement, le kit de diagnostic ou de traitement selon la présente invention comprend :

- dans un 1er compartiment, un 1er composé capable de reconnaître ou de cibler un site pathologique et conjugué avec un des deux peptides selon l'invention ou un précurseur d'un tel composé ; et
- dans un 2nd compartiment, un 2nd composé facilement détectable (pour le kit de diagnostic) ou cytotoxique (pour le kit de traitement) marqué par l'autre peptide selon la présente invention.

Le kit selon la présente invention peut donc comprendre :

- un 1er composé capable de reconnaître ou de cibler un site pathologique et conjugué avec le peptide Jo, un dérivé ou un fragment de celui-ci ou un précurseur d'un tel composé et un 2nd composé facilement détectable marqué par le peptide In, un dérivé ou un fragment de celui-ci ; ou
- un 1er composé capable de reconnaître ou de cibler un site pathologique et conjugué avec le peptide Jo, un dérivé ou un fragment de celui-ci ou un précurseur d'un tel composé et un 2nd composé cytotoxique marqué par le peptide In, un dérivé ou un fragment de celui-ci ; ou
- un 1er composé capable de reconnaître ou de cibler un site pathologique et conjugué avec le peptide In, un dérivé ou un fragment de celui-ci ou un précurseur d'un tel composé et un 2nd composé facilement détectable marqué par le peptide Jo, un dérivé ou un fragment de celui-ci ; ou
- un 1er composé capable de reconnaître ou de cibler un site pathologique et conjugué avec le peptide In, un dérivé ou un fragment de celui-ci ou un précurseur d'un tel composé et un 2nd composé cytotoxique marqué par le peptide Jo, un dérivé ou un fragment de celui-ci.

Un composé capable de reconnaître ou de cibler un site pathologique peut être un anticorps ou un fragment d'anticorps capable de reconnaître un marqueur présent à la surface de cellules pathologiques telles que des cellules cancéreuses. Ce composé peut également être un ligand d'un tel marqueur présent à la surface de cellules pathologiques. En variante encore, ce composé peut être une protéine membranaire capable d'être exprimée dans les cellules pathologiques. De fait, le précurseur de ce composé peut être un acide nucléique codant une protéine de fusion correspondant à cette protéine et au peptide selon l'invention, auquel elle est fusionnée.

Les composés présents dans les deux compartiments du kit selon l'invention peuvent être administrés de façon simultanée ou l'un après l'autre. Les kits selon l'invention peuvent être utilisés aussi bien *in vive qu' in vitro* et notamment sur des échantillons biologiques préalablement issus d'un système vivant, humain ou animal.

La présente invention concerne en outre l'utilisation d'un composé selon la présente invention et notamment d'un composé conjugué avec un seul peptide selon l'invention pour cristalliser ledit composé.

Cette utilisation est fondée sur la propriété des peptides Jo et In associés entre eux à cristalliser dans des conditions robustes et permettant d'obtenir des cristaux avec une bonne qualité de diffraction, comme précédemment explicité. Une telle propriété permet d'envisager de former des cristaux à partir de composés habituellement difficiles à cristalliser tels que des protéines et notamment des protéines membranaires.

Le procédé pour cristalliser un composé selon la présente invention consiste à :

- conjuguer ledit composé avec un des deux peptides selon la présente invention (i.e. avec soit le peptide Jo, un dérivé ou un fragment de celui-ci, soit le peptide In, un dérivé ou un fragment de celui-ci) ;
- mettre le composé ainsi conjugué en présence de l'autre peptide selon la présente invention (i.e. soit le peptide In, un dérivé ou un fragment de celui-ci, si le composé est conjugué avec le peptide Jo, un dérivé ou un fragment de celui-ci ; soit le peptide Jo, un dérivé ou un fragment de celui-ci, si le composé est conjugué avec le peptide In, un dérivé ou un fragment de celui-ci) ;
- soumettre le composé ainsi obtenu à des conditions permettant sa cristallisation.

Le composé qui est obtenu lors de la 2ième étape correspond à un composé conjugué avec un des deux peptides selon l'invention, un fragment ou un dérivé de celui-ci et présentant l'autre peptide selon l'invention, un fragment ou un dérivé de celui-ci associé au 1er peptide via une liaison isopeptidique.

Lorsque le composé selon l'invention est conjugué à un peptide selon l'invention ou à deux peptides selon l'invention, des structures circulaires peuvent être obtenues. La présente invention concerne donc une structure circulaire comprenant au moins un composé tel que précédemment défini.

En effet, lorsque le composé selon l'invention est un composé de type oligonucléotide de formule (I) directement ou indirectement conjugué à un peptide selon l'invention, ce dernier peut présenter une circularisation intra-moléculaire.

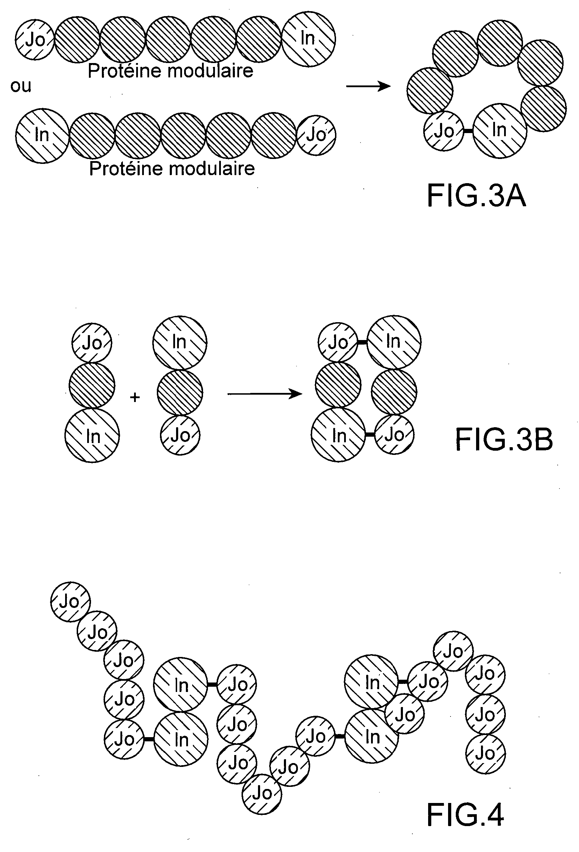

Une circularisation intra-moléculaire peut également être obtenue dans le cadre d'un composé de type protéine grande ou flexible, conjugué à son extrémité N-terminale, directement ou indirectement, avec un des deux peptides selon l'invention et à son extrémité C-terminale, directement ou indirectement, avec l'autre peptide selon l'invention. La Figure 3A est une représentation d'une circularisation intra-moléculaire.

En variante, lorsque le composé selon l'invention est conjugué à son extrémité N-terminale, directement ou indirectement, avec un des deux peptides selon l'invention et à son extrémité C-terminale, directement ou indirectement, avec l'autre peptide selon l'invention, une circularisation inter-moléculaire peut être obtenue. C'est notamment le cas lorsque le composé mis en oeuvre ne présente pas une longueur suffisante et/ou à cause de contraintes stériques trop importantes. La Figure 3B est une représentation d'une circularisation inter-moléculaire qui met en oeuvre deux composés selon l'invention identiques.

Lorsque le composé selon l'invention est conjugué à un peptide selon l'invention ou à deux peptides selon l'invention, des structures multimériques peuvent être obtenues.

La présente invention concerne donc une structure multimérique comprenant au moins deux et avantageusement une pluralité de composés, identiques ou différents, chaque composé étant lié à au moins un autre composé par une liaison isopeptidique. La liaison isopeptidique implique, de fait, un peptide Jo, un dérivé ou un fragment de celui-ci conjugué à un des composés et un peptide In, un dérivé ou un fragment de celui-ci conjugué à un autre des composés.

Tous les cas de figure de composés conjugués par deux peptides selon l'invention tels que précédemment définis et listés sont envisageables pour cette utilisation, la seule contrainte étant qu'un composé soit conjugué par le peptide Jo, un dérivé ou un fragment de celui-ci et un autre composé, identique au ou différent du 1er, soit conjugué par le peptide In, un dérivé ou un fragment de celui-ci et ce, pour qu'une liaison isopeptidique puisse être obtenue. La multimérisation qui est obtenue en mettant en contact ces différents composés est une multimérisation par polymérisation.

La structure multimérique ainsi obtenue peut résulter en une reconstitution ou en un couplage de fonctions, notamment lorsque le ou les composés mis en oeuvre sont des composés de nature peptidique, et proposer des sites multiples d'interactions. Cette structure permet d'augmenter l'avidité d'une interaction donnée.

A titre d'exemples particuliers de telles structures multimériques, on peut citer :

- une structure dans laquelle un seul composé est mis en oeuvre et ce dernier est un antigène ;
- une structure dans laquelle plusieurs composés sont mis en oeuvre et sont des antigènes différents ; une telle structure présentant un intérêt particulier dans le domaine de la vaccination puisqu'elle permet une présentation des antigènes optimisée ;
- une structure dans laquelle plusieurs composés sont mis en oeuvre et sont des monomères différents ; une telle structure présentant un intérêt particulier dans le domaine de la polymérisation puisqu'elle permet d'obtenir de nouveaux polymères qui n'auraient pu être obtenus différemment lorsque sont utilisés des monomères incapables de polymériser ensemble.

Une multimérisation peut également être obtenue en utilisant des composés de type oligopeptide de formule (I) directement ou indirectement conjugué à un seul des peptides selon l'invention. La Figure 4 est une représentation de la structure multimère pouvant être obtenue en utilisant un composé de formule (I) avec m = 0 et n = r = 1 directement conjugué avec le peptide In et un composé de formule (I) avec m = 5, n = 0 et r = 1 directement conjugué avec le peptide Jo.

Les structures circulaires et multimères selon l'invention peuvent être utilisées pour préparer des anticorps.

D'autres caractéristiques et avantages de la présente invention apparaîtront encore à l'homme du métier à la lecture des exemples ci-dessous donnés à titre illustratif et non limitatif, en référence aux figures annexées.

## BRÈVE DESCRIPTION DES DESSINS

La Figure 1 présente l'utilisation des peptides selon l'invention comme colle moléculaire pour assembler deux surfaces (Figure 1A) ou deux composés de type protéique (Figures 1B, 1C et 1D). La Figure 1B et la Figure 1C présentent deux complexes différents pouvant être obtenus selon que le peptide Jo est fusionné à l'extrémité C-terminale ou N-terminale d'une des protéines. La Figure 1D est une représentation du complexe obtenu en utilisant, d'une part, un peptide Jo fusionné en N-terminal à une 1ère protéine et en C-terminal à une 2nde protéine et, d'autre part, un peptide In fusionné en N-terminal à une 3ème protéine et en C-terminal à une 4ème protéine.

La Figure 2 est une schématisation de l'utilisation de la présente invention pour purifier un composé ou pour préparer une biopuce ou un biocapteur.

La Figure 3 présente des structures circulaires pouvant être obtenues avec les peptides selon l'invention via une circularisation intra-moléculaire (Figure 3A) ou inter-moléculaire (Figure 3B).

La Figure 4 présente un type particulier de structure multimérique pouvant être obtenue avec les peptides selon l'invention.

La Figure 5 présente l'expression des peptides Jo, In ou en complexe HisJoIn. Les masses moléculaires indiquées en regard des protéines recombinantes sont calculées.

La Figure 6 présente la purification du complexe HisJoIn.

La Figure 7 présente la formation du complexe HisJoHisIn. Les volumes des protéines HisJo et HisIn utilisés sont indiqués. Le complexe HisJoIn est déposé sur le gel comme contrôle.

La Figure 8 présente la purification du complexe FusJoIn.

La Figure 9 présente les résidus Lys191 et Asn695 forment la liaison covalente entre Jo et In. Les variants du peptide In migrent sous forme de triplet, la migration du mutant D600A est altérée par rapport à celle de la protéine native et du mutant N695A. Le taux de production du mutant Jo-Lys191 est diminué par rapport à celui de la forme Jo native.

La Figure 10 présente des cristaux du complexe HisJoIn. La structure cristallographique du complexe Jo/In isolé et reconstitué *in vitro* a été réalisée (non montré).

La Figure 11 présente la résistance du complexe JoIn à la dénaturation par la chaleur, l'urée et les variations de pH.

La Figure 12 présente un gel d'électrophorèse de la protéine Jo-B1CK-In purifiée par chromatographie d'affinité sur colonne de NiNTA et analysée par SDS-PAGE en absence (-) et en présence (+) de 25 mM de DTT.

La Figure 13 montre que la masse moléculaire apparente du pic entre les fractions 19 et 21 correspond à celle d'une protéine globulaire utilisée pour la calibration de la colonne (Filtration sur gel).

La Figure 14 présente la détection des protéines CK et Jo-B1CK-In par Western blot par anticorps polyclonaux dirigés contre Jo-B1CK-In

## EXPOSÉ DÉTAILLÉ DE MODES DE RÉALISATION PARTICULIERS

### I. Production et purification du complexe « JoIn ».

I.1. Matériels et Méthodes.

A. Séquences des gènes et protéines de dérivés de Jo et de In.

*Séquence dérivée* de *Jo : Séquence nucléotidique*
TCTGACCAGTATCCACAAACAGGGACTTATCCAGATGTTCAAACACC
TTATCAGATTATTAAGGTAGATGGTTCGGAAAAAAACGGACAGCACAAGGCGTTG
AATCCGAATCCATATGAACGTGTGATTCCAGAAGGTACACTTTCAAAGAGAATTT
ATCAAGTGAATAATTTGGATGATAACCAATATGGAATCGAATTGACGGTTAGTGG    GAAAACAGTGTATGAACAAAAAGAT (SEQ ID NO: 5 du listage de séquences en annexe)

*Séquence dérivée de Jo : Séquence en acides aminés*
SDQYPQTGTYPDVQTPYQIIKVDGSEKNGQHKALNPNPYERVIPEGT    LSKRIYQVNNLDDNQYGIELTVSGKTVYEQKD (SEQ ID NO: 6 du listage de séquences en annexe)

*Séquence dérivée de In : Séquence nucléotidique* ACTGAAAAGAAATCAATTGAGAATGGTACGATTACAGATC-
CGATGGG                TGAGTTAATTGATTTGCAATTGGGCACAGATGGAAGATTTGATCCAGCAGATTAC
ACTTTAACTGCAAACGATGGTAGTCGCTTGGAGAATGGACAAGCTGTAGGTGGTC
CACAAAATGATGGTGGTTTGTTAAAAAATGCAAAGTGCTCTATGATACGACTGA
GAAAAGGATTCGTGTAACAGGTCTGTACCTTGGAACGGATGAAAAAGTTACGTTG
ACCTACAATGTTCGTTTGAATGATGAGTTTGTAAGCAATAAATTTTATGATACCA
ATGGTCGAACAACCTTACATCCTAAGGAAGTAGAACAGAACACAGTGCGCGACTT                CCCGATTCCTAA-
GATTCGTGAT (SEQ ID NO: 7 du listage de séquences en annexe)

*Séquence dérivée de In : Séquence en acides aminés*
TEKKSIENGTITDPMGELIDLQLGTDGRFDPADYTLTANDGSRLENG QAVGGPQNDGGLLKNAKVLYDTTEKRIRVTGLYLGTDEKVTLTYNVRLNDEFVSN KFYDTNGRTTLHPKEVEQNTVRDFPIPKIRD (SEQ ID NO : 8 du listage de séquences en annexe)

B. Clonage des gènes codant pour des dérivés de Jo et de In dans des vecteurs bi-cistroniques.

Les gènes codant pour les séquences dérivées de Jo et de In ont été amplifiés par PCR à partir d'ADN génomique de la souche de *S. pneumoniae* TIGR4. Les fragments de gène codant pour des dérivés de Jo et de In ont été clonés par enzymes de restriction dans le vecteur pETDuet (Novagen), respectivement dans les MCSI et II : soit dans le même plasmide (co-expression des peptides Jo et In) soit dans des vecteurs distincts permettant l'expression de Jo et In de façon indépendante ; le clonage du gène codant pour Jo dans le MCSI du vecteur pETDuet conduit à la fusion en N-terminal d'une étiquette 6xHis. Le gène codant pour In en fusion avec une étiquette 6xHis est cloné dans le vecteur pACYCDuet dans le site MCSI. La fusion des gènes Jo et In a été effectuée par mutagenèse dirigée (Stratagene).

C. Vecteurs construits.

pETDuet-HisJoIn co-expression des dérivés de Jo (His tag en N-ter) et de In
pETDuet-HisJo expression d'un dérivé de Jo (His tag en N-ter)
pETDuet-In expression d'un dérivé de In pACYCDuet-HisIn expression d'un dérivé de In (His tag en N-ter)
pETDuet-FusJoIn fusion des dérivés de Jo (His tag en N-ter) et de In

D. Production des protéines et complexes.

La souche d'E. *coli* BL21(DE3)Star est transformée par les vecteurs construits et indiqués ci-dessus. Chaque culture transformée avec le plasmide pETDuet contient de l'ampicilline à 100 µg/ml et avec 34 µg/ml de chloramphénicol lorsque le plasmide pACYCDuet est utilisé. Une pré-culture de 50 ml de milieu LB est inoculée et incubée 16 h à 37°C sous agitation puis est utilisée pour inoculer 500 ml de milieu LB. La culture bactérienne s'effectue à 37°C sous agitation pendant 2 h puis une solution d'IPTG à 0,5 mM finale est ajoutée afin d'induire l'expression des peptides dérivés de Jo et de In.

Afin de tester l'expression des protéines recombinantes, un volume de 5 µl des cultures induites est prélevé, additionné de 5 µl de solution dénaturante dite « Laemmli » tamponnée à pH 8.0, contenant un détergent (SDS), un agent réducteur (béta-mercaptoéthanol), du glycérol et du bleu de bromophénol, puis chauffé 5 min à 100°C avant dépôt sur SDS-PAGE 15%.

E. Purification des protéines et complexes.

Les cultures bactériennes induites sont centrifugées à 4000 rpm pendant 10 min. Le culot bactérien est mis en suspension dans un tampon composé par 50 mM Tris pH 8.0, 200 mM NaCl, 20 mM imidazole (tampon A) additionné de deux pastilles d'inhibiteurs de protéases, Complete-EDTA free (Roche). La lyse bactérienne est effectuée par sonication pendant 4 min puis soumise à une centrifugation à 18000 rpm pendant 20 min afin de récupérer la fraction soluble contenant les peptides Jo et In. Celle-ci est déposée sur une colonne chromatographique HisTrap de 1 ml équilibrée dans le tampon A, afin de purifier les peptides recombinantes dérivés de Jo et de In fusionnées à l'étiquette His6x. L'élution des protéines individuelles ou en complexe est effectuée par des concentrations croissantes d'imidazole. Les protéines sont analysées sur SDS-PAGE 15%. De façon générale, la production recombinante de dérivés de Jo et de In sous forme soluble chez E. coli, seuls ou en complexe, présente un fort rendement (entre 50 et 100 mg de complexe soluble purifié par litre de culture) rendant les coûts de production minimes et la mise à l'échelle pratique.

I.2. Résultats.

A. Formation du complexe JoIn *in vivo* dans *E. coli.*

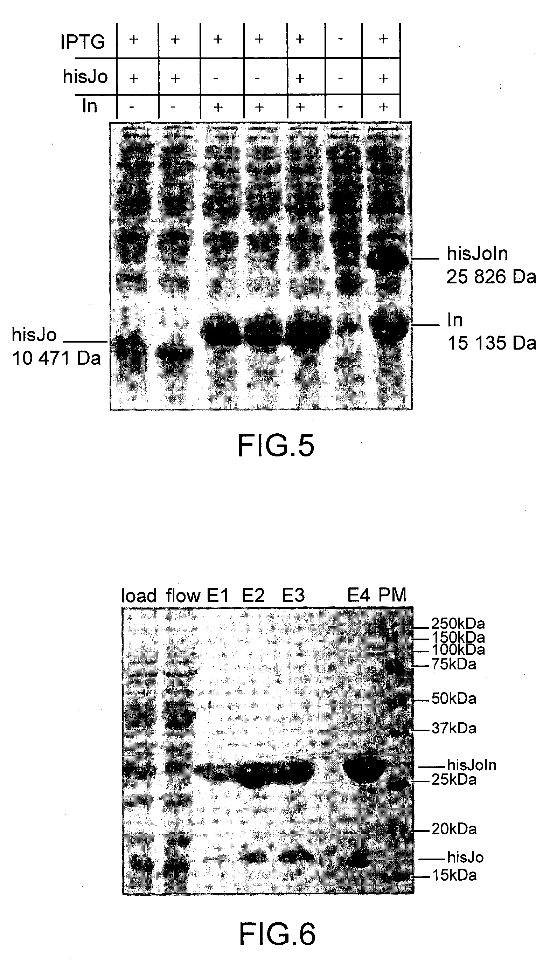

Les souches transformées avec les vecteurs pETDuet-HisJo (expression de HisJo), pETDuet-In (expression de In) et pETDuet-HisJoIn (co-expression de HisJo et In) ont été utilisées. L'expression des dérivés des peptides Jo ou In seuls ou formant le complexe HisJoIn est observé dans la Figure 5.

B. Purification du complexe JoIn.

Le complexe HisJoIn produit lors de la co-expression de HisJo et In est purifié sur colonne HisTrap (GE Healthcare Life Sciences). Le résultat obtenu est présenté dans la Figure 6.

C. Formation du complexe JoIn à partir des protéines Jo et In purifiées.

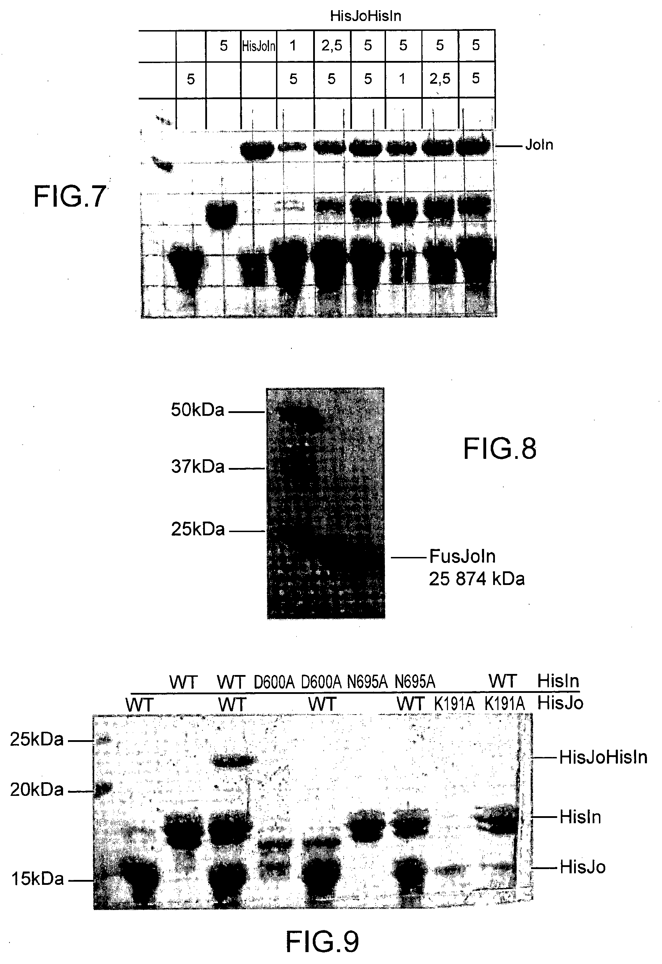

Les souches transformées avec les vecteurs pETDuet-HisJo (expression de HisJo) et pACYCDuet-HisIn (expression de HisIn) ont été utilisées. Les protéines HisJo et HisIn ont été purifiées séparément et incubées ensemble, à des ratios différents comme indiqué dans la Figure 7, pendant 12 min à température ambiante avant d'être analysé sur SDS-PAGE.

Des expériences complémentaires de cinétique ont été effectuées. L'incubation à température ambiante ou à 37°C ne modifie pas la cinétique de formation du complexe.

D. Purification du complexe FusJoIn.

Les gènes codant pour HisJo et In ont été fusionnés via un linker codant pour la séquence GSTPGSV. La production et la purification du complexe FusJoIn ont été effectuées selon les protocoles décrits précédemment (Figure 8).

II. **Caractérisation du complexe JoIn.**

II.1. Analyse par spectrométrie de masse en mode électrospray.

Les protéines purifiées HisJo et HisIn ainsi que les complexes purifiés HisJoIn, HisJoHisIn et FusJoIn ont été analysés par spectrométrie de masse en mode électrospray. La formation de la liaison intramoléculaire covalente entre la Lys191 et l'Asn695 s'accompagne de la perte d'un ion $NH_3$, soit d'une masse mesurée ayant perdu 17 Da par rapport à la masse théorique. L'ensemble des données expérimentales obtenues est présenté ci-dessous.

*HisJo* :

```
GSSHHHHHHSQDPSDQYPQTGTYPDVQTPYQIIKVDGSEKNGQHKAL

NPNPYERVIPEGTLSKRIYQVNNLDDNQYGIELTVSGKTVYEQKD    (SEQ    ID

NO: 9 du listage de séquences en annexe)
```

|  |  |
|---|---|
| Masse mesurée par ESI-MS | 10471 |
| Masse calculée | 10470,32 |

*HisIn* :

```
GSSHHHHHHSQDPTEKKSIENGTITDPMGELIDLQLGTDGRFDPADY

TLTANDGSRLENGQAVGGPQNDGGLLKNAKVLYDTTEKRIRVTGLYLGTDEKVTL

TYNVRLNDEFVSNKFYDTNGRTTLHPKEVEQNTVRDFPIPKIRD    (SEQ    ID

NO: 10 du listage de séquences en annexe)
```

|  |  |
|---|---|
| Masse mesurée par ESI-MS | 16319 |
| Masse calculée | 16317,9 |

*HisJoIn* :

```
        GSSHHHHHHSQDPSDQYPQTGTYPDVQTPYQIIKVDGSEKNGQHKAL
NPNPYERVIPEGTLSKRIYQVNNLDDNQYGIELTVSGKTVYEQKDADLTEKKSIE
NGTITDPMGELIDLQLGTDGRFDPADYTLTANDGSRLENGQAVGGPQNDGGLLKN
AKVLYDTTEKRIRVTGLYLGTDEKVTLTYNVRLNDEFVSNKFYDTNGRTTLHPKE
VEQNTVRDFPIPKIRD (SEQ ID NO: 11 du listage de séquences
en annexe)
```

|  |  |
|---|---|
| Masse mesurée par ESI-MS | 25589 |
| Masse calculée | 25606,03 |

**Formation du pont intramoléculaire covalent**

*HisJoHisIn* :

```
        GSSHHHHHHSQDPSDQYPQTGTYPDVQTPYQIIKVDGSEKNGQHKAL
NPNPYERVIPEGTLSKRIYQVNNLDDNQYGIELTVSGKTVYEQKDMGSSHHHHHH
SQDPTEKKSIENGTITDPMGELIDLQLGTDGRFDPADYTLTANDGSRLENGQAVG
GPQNDGGLLKNAKVLYDTTEKRIRVTGLYLGTDEKVTLTYNVRLNDEFVSNKFYD
TNGRTTLHPKEVEQNTVRDFPIPKIRD (SEQ ID NO: 12 du listage
de séquences en annexe)
```

|  |  |
|---|---|
| Masse mesurée par ESI-MS | 26772 |
| Masse calculée | 26788,16 |

**Formation du pont intramoléculaire covalent**

*FusJoIn :*

```
        GSSHHHHHHSQDPSDQYPQTGTYPDVQTPYQIIKVDGSEKNGQHKAL
NPNPYERVIPEGTLSKRIYQVNNLDDNQYGIELTVSGKTVYEQKDGSTPGSVTEK
KSIENGTITDPMGELIDLQLGTDGRFDPADYTLTANDGSRLENGQAVGGPQNDGG
LLKNAKVLYDTTEKRIRVTGLYLGTDEKVTLTYNVRLNDEFVSNKFYDTNGRTTL
HPKEVEQNTVRDFPIPKIRD (SEQ ID NO: 13 du listage de
séquences en annexe)
```

| Masse mesurée par ESI-MS | 25857 |
| Masse calculée | 25874 |

**Formation du pont intramoléculaire covalent**

II.2. Mutagenèse des résidus impliqués dans la formation du pont intramoléculaire covalent.

Dans le cadre de l'étude de la protéine RrgA, les résidus impliqués dans la formation du pont intramoléculaire du domaine D2 entre les régions H1 (ici nommé Jo) et H2 (ici nommé In), soit la Lys191, l'Asn695 et l'Asp600 avaient été mutés en Ala ce qui conduisait à l'abolition de la formation du pont covalent.

Les inventeurs ont voulu vérifier ici si ces mutations, portées par les protéines HisJo et HisIn pouvaient également empêcher la formation du pont isopeptidique. Ces mutations ont été introduites dans les constructions codant pour HisJo et HisIn. Les protéines natives (ou wild-type, WT) et mutées ont été purifiées puis testées en combinaison pour la formation du complexe.

Les résultats présentés dans la Figure 9 montrent que la formation du pont covalent entre Jo et In ne s'établit qu'avec les formes natives, indiquant que les résidus Lys191, Asn695 et Asp600 interviennent dans la formation du pont covalent également dans des conditions *in vitro.*

II.3. Résolution de la structure cristallographique du complexe HisJoIn.

Des cristaux ont été obtenus par la méthode des gouttes suspendues dans les conditions suivantes en l'espace de 3-4 jours :

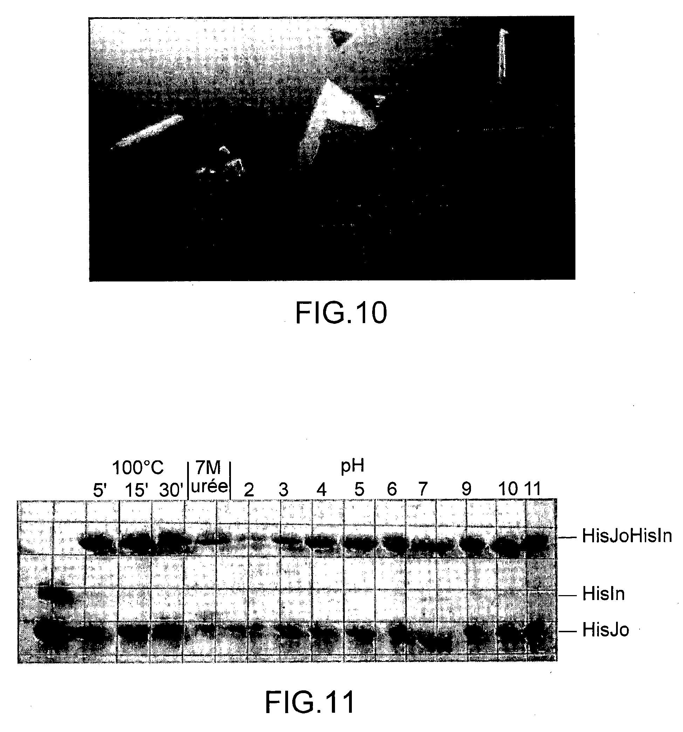

- concentration de protéine : 23 mg/ml
- solution de cristallisation : 1,6 M ammonium sulfate, 0,1 M Acide citrique pH 4,0
- température : 20°C (Figure 10).

Des données de diffraction à 2 Å ont été collectées à l'ESRF (Grenoble). La résolution de la structure est en cours de réalisation par remplacement moléculaire.

III. **Stabilité du complexe JoIn.**

La liaison covalente établie spontanément entre les peptides Jo et In stabilise le complexe JoIn.

Les inventeurs ont montré que le complexe covalent établi entre les peptides dérivés de Jo et de In est résistant à la dénaturation par la chaleur, par un agent chaotrope, par variations de pH entre 2 et 11, à l'action de détergents (non montré). Le complexe HisJoHisIn a été incubé pendant 30 min ou 1 h en présence de ces différents agents à température ambiante avant d'être analysé par SDS-PAGE. Les formes HisJo et HisIn sont déposées sur gel comme contrôle (Figure 11).

Le complexe formé entre les peptides dérivés de Jo et de In est résistant à la digestion protéolytique par la trypsine dans des rapports de concentration de 1 : 1 (gel non montré).

IV. **Utilisation du complexe JoIn dans l'ingénierie des protéines circulaires.**

Cette application spécifique du complexe JoIn concerne la production de fragments solubles de protéines membranaires en vue d'études fonctionnelles et structurales et de production d'antigènes solubles.

IV.1. Contexte et objectifs.

Des régions solubles (en général des boucles) exposées par des protéines membranaires peuvent jouer un rôle fonctionnel et/ou structural (par exemple multimérisation).

La fusion d'une telle boucle au complexe covalent Jo-In, mimant ainsi les régions transmembranaires, peut favoriser la structuration, l'exposition et la production de ces régions. De plus, ces protéines de fusion sont solubles, constituent des antigènes plus faciles à produire et à purifier, dont l'antigénicité peut être accrue en comparaison du contexte de la protéine native membranaire.

D'autre part, ce mode de production d'antigène présente un coût moindre en comparaison de la synthèse de peptides. L'antigène circularisé par l'ajout à ses extrémités N- et C-terminales de Jo et de In (ou l'inverse) est attendu comme étant plus stable qu'un peptide isolé.

De plus, la présence de Jo et In dans la séquence facilite la production en abondance d'une protéine soluble en quantité abondante chez *E. coli.* Il est aussi possible que la présence de deux fragments de protéines issus d'une bactérie pathogène (le pneumocoque) joue le rôle d'un adjuvant stimulant la production d'anticorps.

L'exemple ci-après est un exemple d'application portant sur l'expression d'une boucle de 56 acides aminés (fragment de protéine nommée BC1K) appartenant à un canal potassique (CK) dont la topologie prédite inclus 4 régions transmembranaires.

IV.2. Matériels et méthodes.

A. *Construction d'un vecteur pour la production de protéines circulaires.*

Le plasmide dérivé de pET-Duet-1 (Novagen) contenant les séquences nucléotidiques (...**cggttagtgggaaaacagtgtatgaacaaaaagat**ggttctaccccgggttct gtt*actgaaaagaaatcaattgagaatggtacg*...) (SEQ ID NO: 14 dans le listage de séquences annexé) codant pour un dérivé de Jo (séquence partielle en gras) et un dérivé de In (séquence partielle en italique) séparées par un linker de 7 acides aminés GSTPGSV (SEQ ID NO: 15 dans le listage de séquences annexé) a été modifié par mutagenèse dirigée en utilisant les oligonucléotides de synthèse Mut 1 et Mut 2 (Tableau 1) pour obtenir le vecteur d'expression Jo-MCS1-In.

Tableau 1 :

| Oligo 1 | 5' CGGTTAGTGGGAAAACAGTGTATGAACAAAAAGATGGAATTCGGTACCCCT GCAGGGAGCTGAAGCTTACTGAAAAGAAATCAATTGAGAATGGTACG<br>(SEQ ID NO: 16 dans le listage de séquences annexé) |
|---|---|
| Oligo 2 | 5' CGTACCATTCTCAATTGATTTCTTTTCAGTAAGCTTCAGCTCCCTGCAGGG GTACCGAATTCCATCTTTTTGTTCATACACTGTTTTCCCACTAACCG<br>(SEQ ID NO: 17 dans le listage de séquences annexé) |
| Oligo 3 | 5'GCGCGAATTCCAGAAATTTCTCAGCGTACG<br>(SEQ ID NO: 18 dans le listage de séquences annexé) |
| Oligo 4 | 5'GCGCAAGCTTGCTGATTTGGTTTGATG<br>(SEQ ID NO: 19 dans le listage de séquences annexé) |

B. *Clonage de B1CK dans le vecteur Jo-MCS1-In.*

La séquence codant pour B1CK est amplifiée par PCR en utilisant les Oligos 3 et 4 (Tableau 1). Ce fragment de PCR est purifié et cloné en utilisant le kit PCR-Script Amp Cloning (Agilent). Le vecteur PCR-Script Amp-B1CK est digéré simultanément par les enzymes de restrictions EcoRI et HindIII pour produire un fragment de 167 nucléotides. Ce fragment purifié sur gel d'agarose est ensuite ligué dans le vecteur Jo-MCS1-In pré-clivés par EcoRI et HindIII et le produit de ligation est transformé dans la souche d'E. *coli* DH5alpha. Le vecteur recombinant purifié à partir d'un clone transformé est nommé Jo-B1CK-In.

C. *Expression dans E. coli et purification de B1CK.*

Le vecteur Jo-B1CK-In est transformé dans la souche BL21(DE3)Star (Invitrogen). La souche transformée est cultivée dans 1 litre de milieu de culture LB à 37°C jusqu'à une DO$_{600}$ de 0.6 pour être ensuite induite par 0,5 mM d'IPTG pendant une nuit à 20°C. La culture bactérienne est centrifugée pendant 10 min à 5000 g et le culot bactérien est mis en suspension dans 50 mL de tampon 50 mM Tris pH : 7,5, NaCl 150 mM, imidazole 25 mM. Les bactéries sont lysées par un traitement de 3 cycles au microfluidizer de 10000 psi. Le lysat est centrifugé pendant 25 min à 40000 g.

Le surnageant est chargé sur une colonne de 1 mL de NiNTA (GE Healthcare), lavée par 100 mL de tampon 100 mM Tris pH : 7,5, NaCl 150 mM, imidazole 50 mM. Les protéines sont éluées par un gradient d'imidazole de 50 mM à 300 mM dans le tampon 50 mM Tris pH : 7,5, NaCl 150 mM. Les fractions contenant le pic d'absorption à 280 nm sont rassemblées et concentrées 20 à 50 fois en utilisant un centricon Amicon Ultra 10K (Millipore). Les protéines sont directement utilisées pour la production d'anticorps chez le lapin (Covalab) après dialyse contre un volume de 100 fois de tampon phosphate 20 mM NaCl 50 mM pH 7,5.

L'analyse du degré de pureté, d'homogénéité en solution et du degré d'oligomérisation de la protéine de fusion est réalisée par SDS-PAGE en présence ou en absence de 25 mM dithiothreithol et par chromatographie d'exclusion sur gel en utilisant une colonne S200 10/300GL (GE Healthcare).

IV.3. Résultats.

A. *Construction d'un vecteur pour la production de protéines circulaires.*

Les sites de restriction successivement dans la séquence, EcoRI, KpnI, PstI, SacI et HindIII ont été insérés par mutagenèse dirigée entre les séquences codantes pour Jo et In.

TTTTGTTTAACTTTAAGAAGGAGATATACCATGGGCAGCAGC*CATCA*
*CCATCATCACCAC*AGCCAGGATCCG**TCTGACCAGTATCCACAAACAGGGACTTAT**
**CCAGATGTTCAAACACCTTATCAGATTATTAAGGTAGATGGTTCGGAAAAAAACG**
**GACAGCACAAGGCGTTGAATCCGAATCCATATGAACGTGTGATTCCAGAAGGTAC**
**ACTTTCAAAGAGAATTTATCAAGTGAATAATTTGGATGATAACCAATATGGAATC**
**GAATTGACGGTTAGTGGGAAAACAGTGTATGAACAAAAGAT**GGAATTCGGTACC
CCTGCAGGGAGCTGAAGCTT*ACTGAAAAGAAATCAATTGAGAATGGTACGATTAC*
*AGATCCGATGGGTGAGTTAATTGATTTGCAATTGGGCACAGATGGAAGATTTGAT*
*CCAGCAGATTACACTTTAACTGCAAACGATGGTAGTCGCTTGGAGAATGGACAAG*
*CTGTAGGTGGTCCACAAAATGATGGTGGTTTGTTAAAAAATGCAAAGTGCTCTA*
*TGATACGACTGAGAAAAGGATTCGTGTAACAGGTCTGTACCTTGGAACGGATGAA*
*AAAGTTACGTTGACCTACAATGTTCGTTTGAATGATGAGTTTGTAAGCAATAAAT*
*TTTATGATACCAATGGTCGAACAACCTTACATCCTAAGGAAGTAGAACAGAACAC*
*AGTGCGCGACTTCCCGATTCCTAAGATTCGTGATTGA*CTCGAGTCTGGT......

La séquence ci-dessus (SEQ ID NO: 20 dans le listage de séquences en annexe) correspond à la séquence nucléotidique de la région pertinente du vecteur de clonage permettant l'expression d'une protéine de fusion ayant les protéines Jo et In localisées respectivement en N et C-terminus de la protéine. En italique souligné : séquence nucléotidique codant pour 6 histidines ; en gras : séquence nucléotidique codant pour un dérivé de Jo ; en italique : séquence nucléotidique codant pour un dérivé de In ; et entre la séquence nucléotidique codant pour le dérivé de Jo et celle codant pour le dérivé de In, le site de clonage multiple (5 sites de restriction soulignés). L'expression de la protéine de fusion est sous le contrôle du promoteur T7Lac.

B. *Clonage de la boucle 1 de CK (B1CK) dans le vecteur « Jo-In ».*

La séquence codant pour la boucle B1CK a été insérée entre les séquences Jo et In.

ATGGGCAGCAGC*CATCACCATCATCACCAC*AGCCAGGATCCG**TCTGA CCAGTATCCACAAACAGGGACTTATCCAGATGTTCAAACACCTTATCAGATTATT AAGGTAGATGGTTCGGAAAAAACGGACAGCACAAGGCGTTGAATCCGAATCCAT ATGAACGTGTGATTCCAGAAGGTACACTTTCAAAGAGAATTTATCAAGTGAATAA TTTGGATGATAACCAATATGGAATCGAATTGACGGTTAGTGGGAAAACAGTGTAT GAACAAAAAGAT**GGAATTCCAGAAATTTCTCAGCGTACGACCATTGTGATTCAGA AACAAACGTTTATCAGTCAGCATTCCTGTGTGAATAGCACCGAACTGGATGAACT GATCCAACAAATTGTGGCAGCGATCAATGCCGGTATTATTCCACTGGGGAACACA TCAAACCAAATCAGCAAGCTT*ACTGAAAAGAAATCAATTGAGAATGGTACGATTA CAGATCCGATGGGTGAGTTAATTGATTTGCAATTGGGCACAGATGGAAGATTTGA TCCAGCAGATTACACTTTAACTGCAAACGATGGTAGTCGCTTGGAGAATGGACAA GCTGTAGGTGGTCCACAAAATGATGGTGGTTTGTTAAAAAATGCAAAAGTGCTCT ATGATACGACTGAGAAAGGATTCGTGTAACAGGTCTGTACCTTGGAACGGATGA AAAAGTTACGTTGACCTACAATGTTCGTTTGAATGATGAGTTTGTAAGCAATAAA TTTTATGATACCAATGGTCGAACAACCTTACATCCTAAGGAAGTAGAACAGAACA CAGTGCGCGACTTCCCGATTCCTAAGATTCGTGATTGA*CTCGAGTCTGGT

La séquence ci-dessus (SEQ ID NO: 21 dans le listage de séquences en annexe) correspond à la séquence nucléotidique nucléotidique de la région pertinente du vecteur permettant l'expression d'une protéine de fusion ayant les protéines Jo et In localisées respectivement en N et C-terminus de la boucle 1 du canal potassique. En italique souligné : séquence nucléotidique codant pour 6 histidines ; en gras : séquence nucléotidique codant pour un dérivé de Jo ; en italique : séquence nucléotidique codant pour un dérivé de In ; et, entre la séquence nucléotidique codant pour le dérivé de Jo et celle codant pour le dérivé de In, en souligné, séquence nucléotidique codant pour la boucle 1 du canal potassique et, en non souligné, séquences nucléotidiques codant pour des acides aminés du linker.

MGSS*HHHHHH*SQDP**SDQYPQTGTYPDVQTPYQIIKVDGSEKNGQHKA LNPNPYERVIPEGTLSKRIYQVNNLDDNQYGIELTVSGKTVYEQKD**GIPEISQRT TIVIQKQTFISQHSCVNSTLDELIQQIVAAINAGIIPLGNTSNQISKL*TEKKSIE NGTITDPMGELIDLQLGTDGRFDPADYTLTANDGSRLENGQAVGGPQNDGGLLKN AKVLYDTTEKRIRVTGLYLGTDEKVTLTYNVRLNDEFVSNKFYDTNGRTTLHPKE VEQNTVRDFPIPKIRD* Stop

La séquence ci-dessus (SEQ ID NO: 22 dans le listage de séquences en annexe) correspond à la séquence d'acides aminés de la fusion Jo-B1CK-In. En italique souligné : séquence de l'étiquette de 6 histidines ; en gras : séquence d'acides aminés du dérivé de Jo ; en italique : séquence d'acides aminés du dérivé de In ; et, entre la séquence d'acides aminés du dérivé de Jo et celle codant pour le dérivé de In, séquence soulignée d'acides aminés de la boucle 1 du canal potassique et séquence non soulignée d'acides aminés du linker. Nombre total de résidus : 284, masse moléculaire calculée : 31 677,1 Da.

C. *Purification de la protéine Jo-B1CK-In et analyse en présence et en absence d'un agent réducteur.*

La protéine recombinante Jo-B1CK-In purifiée par affinité sur colonne NiNTA est analysée par SDS-PAGE en présence ou non d'un agent réducteur, le DTT.

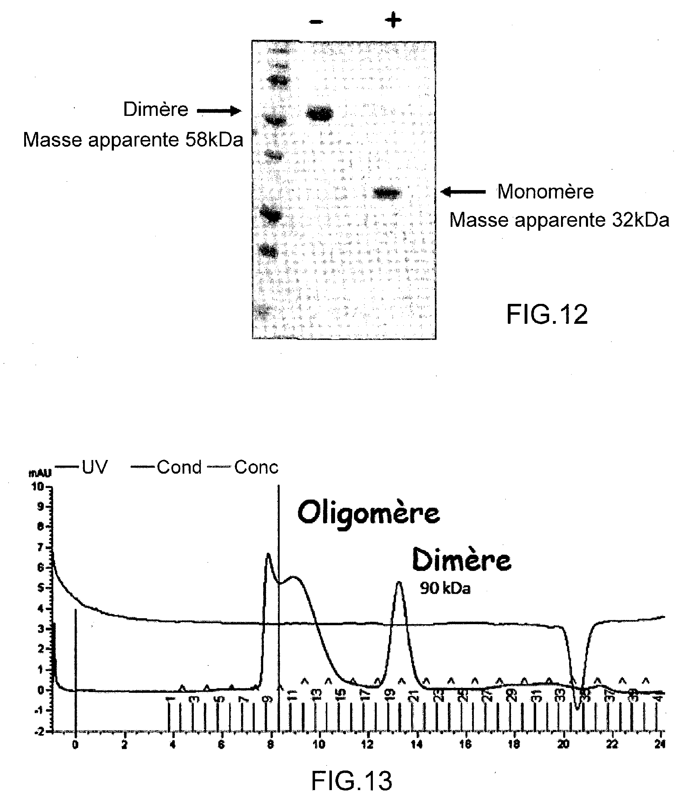

En présence de DTT, la protéine migre avec une masse apparente de 32kDa contre une masse double en absence du réducteur (Figure 12). Des publications récentes décrivant des structures à haute résolution de protéines homologues indiquent que ces canaux dimérisent par un pont disulfure localisé sur une boucle, qui correspond à la boucle B1CK qui contient une cystéine (Miller & Long, 2012, Science, vol. 335, pages 432-436 ; Brohawn et al., 2012, Science, vol. 335, pages 436-441).

La dimérisation par les cystéines de Jo-B1CK-In démontre que la boucle 1 du canal K contrainte à sa base par la protéine Jo-In possède une configuration native. Par conséquent, la plateforme Jo-In mime l'effet des deux régions transmembranaires portant la boucle 1.

Ces résultats ont été confirmés par filtration sur gel. La protéine est éluée comme une protéine globulaire de 90 kDa correspondant au dimère (masse théorique 63 kDa). Aucune trace de monomère n'est détectée. La dimérisation n'est pas due à l'interaction entre deux complexes covalents Jo-In car une Jo-In fusionnée via un linker de 7 acides aminés à la place de la boucle 1, migre sous forme monomérique dans des conditions expérimentales identiques (données non présentées). Le fait que le dimère Jo-B1CK-In migre à travers la colonne avec une masse apparente (90 kDa) supérieure à la masse théorique (63 kDa) pourrait provenir de la mobilité en solution de la boucle. Les espèces de masse moléculaire apparente supérieure (multimères) proviennent probablement de liaison entre Jo et In appartenant à des molécules indépendantes (JoB1CK-In-Jo-B1CK-In circulaire les Jo et In aux extrémités étant liés covalement) liées entre elles par les cystéines de la boucle 1 (Figure 13).

D. *Production d'anticorps chez le lapin.*

La protéine Jo-B1CK-In purifiée a été utilisée pour produire des anticorps polyclonaux chez le lapin (fournisseur Covalab).

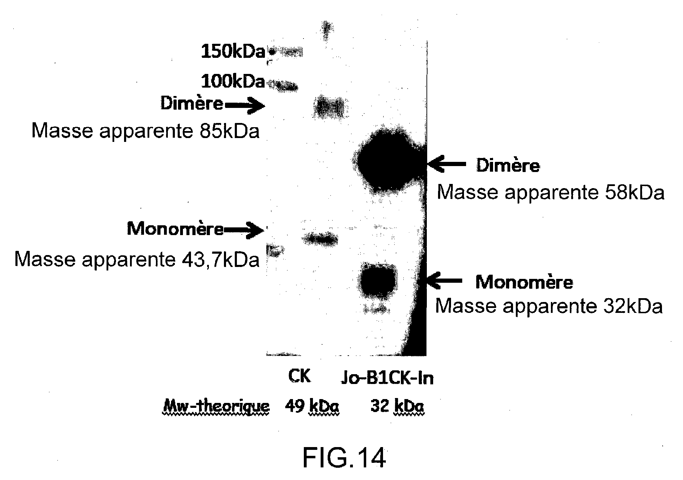

Les sera collectés 53 jours après l'injection ont été testés pour leur capacité à détecter Jo-B1CK-In et le canal K recombinant (CK) (Figure 14). Les sera dilués 10 000 fois reconnaissent efficacement les deux protéines. La plateforme Jo-In est donc efficace pour la production d'anticorps polyclonaux contre des protéines membranaires qui sont souvent difficiles à produire et qui ont des propriétés antigéniques réduites.

## Revendications

1.  Peptide apte à former un complexe covalent consistant en :

    - soit le peptide, désigné peptide Jo, dont la séquence en acides aminés est la suivante :

    QYPQTGTYPDVQTPYQIIKVDGSEKNGQHKALNPNPYERVIPEGTLS   KRIYQVNNLDDNQYGIELTVSGKTVYEQ (SEQ ID NO: 2 dans le listage de séquences en annexe), un dérivé ou un fragment de celui-ci ;

    - soit le peptide, désigné peptide In, dont la séquence en acides aminés est la suivante :

    IENGTITDPMGELIDLQLGTDGRFDPADYTLTANDGSRLENGQAVGG
    PQNDGGLLKNAKVLYDTTEKRIRVTGLYLGTDEKVTLTYNVRLNDEFVSNKFYDT NGRTTLHPKEVEQNTVRDFPIPKIRDVR (SEQ ID NO: 4 dans le listage de séquences en annexe), un dérivé ou un fragment
    de celui-ci,
    lesdits dérivés et fragments étant aptes à former spontanément un complexe covalent au moyen d'une
    liaison isopeptidique,
    un dérivé du peptide Jo et un dérivé du peptide In présentant respectivement au moins 40% d'identité avec
    SEQ ID NO: 2 et SEQ ID NO: 4.

2.  Hétérodimère formé par les deux peptides tels que définis à la revendication 1 et liés l'un à l'autre par une liaison
    isopeptidique.

3.  Polynucléotide isolé, différent du polynucléotide naturel codant la protéine RrgA de *Streptococcus pneumoniae,*
    choisi parmi les différents polynucléotides ci-après :

    i) un polynucléotide codant un peptide tel que défini à la revendication 1 ;

ii) un polynucléotide complémentaire du polynucléotide tel que défini au point (i) ;
iii) un polynucléotide d'au moins 18 nucléotides, capable de s'hybrider dans des conditions de forte stringence aux polynucléotides tels que définis aux points (i) et (ii),

ledit polynucléotide (iii) capable de s'hybrider dans des conditions de forte stringence avec le polynucléotide tel que défini au point (ii) code un peptide apte à former spontanément un complexe covalent au moyen d'une liaison isopeptidique.

**4.** Polynucléotide selon la revendication 3, **caractérisé en ce qu'**il comprend au moins une séquence nucléotidique présentant au moins 40%, au moins 45%, au moins 50%, au moins 55%, au moins 60%, au moins 65%, au moins 70%, au moins 75%, au moins 80%, au moins 85%, au moins 90%, au moins 95% et/ou au moins 99% d'identité avec une des séquences nucléotidiques ci-après ou une de leurs séquences complémentaires :

CAGTATCCACAAACAGGGACTTATCCAGATGTTCAAACACCTTATCA
GATTATTAAGGTAGATGGTTCGGAAAAAAACGGACAGCACAAGGCGTTGAATCCG
AATCCATATGAACGTGTGATTCCAGAAGGTACACTTTCAAAGAGAATTTATCAAG
TGAATAATTTGGATGATAACCAATATGGAATCGAATTGACGGTTAGTGGGAAAAC     AGTGTATGAACAA
(SEQ ID NO: 1 du listage de séquences en annexe) ou
ATTGAGAATGGTACGATTACAGATCCGATGGGTGAGTTAATTGATTT
GCAATTGGGCACAGATGGAAGATTTGATCCAGCAGATTACACTTTAACTGCAAAC
GATGGTAGTCGCTTGGAGAATGGACAAGCTGTAGGTGGTCCACAAAATGATGGTG
GTTTGTTAAAAAATGCAAAAGTGCTCTATGATACGACTGAGAAAAGGATTCGTGT
AACAGGTCTGTACCTTGGAACGGATGAAAAAGTTACGTTGACCTACAATGTTCGT
TTGAATGATGAGTTTGTAAGCAATAAATTTTATGATACCAATGGTCGAACAACCT
TACATCCTAAGGAAGTAGAACAGAACACAGTGCGCGACTTCCCGATTCCTAAGAT   TCGTGAT (SEQ ID NO: 3 du listage de séquences en annexe),
ladite séquence nucléotidique code un peptide apte à former spontanément un complexe covalent au moyen d'une liaison isopeptidique.

**5.** Vecteur de clonage et/ou d'expression contenant au moins un polynucléotide tel que défini à la revendication 3 ou 4.

**6.** Organisme hôte transformé par ou comprenant un polynucléotide tel que défini à la revendication 3 ou 4 ou un vecteur tel que défini à la revendication 5.

**7.** Support solide à la surface duquel un (ou plusieurs) peptide(s) tel(s) que défini(s) à la revendication 1 est(sont) immobilisé(s).

**8.** Composé conjugué avec au moins un peptide tel que défini à la revendication 1.

**9.** Composé selon la revendication 8, **caractérisé en ce que** ledit composé est une molécule biologique ou biologiquement active, un monomère, un composé facilement détectable ou un composé cytotoxique.

**10.** Composé selon la revendication 8 ou 9, **caractérisé en ce que** ledit composé est conjugué par deux peptides, identiques ou différents, tels que définis à la revendication 1.

**11.** Utilisation d'un support solide selon la revendication 7 et d'un composé selon l'une quelconque des revendications 8 à 10, pour purifier ledit composé ou pour préparer une biopuce ou un biocapteur,
ledit composé étant conjugué avec l'un des deux peptides tels que définis à la revendication 1 et l'autre peptide tel que défini à la revendication 1 étant immobilisé sur ledit support solide.

**12.** Procédé pour purifier un composé, comprenant les étapes consistant à :

- conjuguer indirectement ledit composé avec un des deux peptides tels que définis à la revendication 1, un bras espaceur clivable séparant ledit composé et ledit peptide ;
- préparer un support solide à la surface duquel l'autre peptide tel que défini à la revendication 1 est immobilisé ;
- mettre le composé ainsi conjugué en présence du support ainsi préparé, moyennant quoi le composé conjugué est immobilisé sur le support solide ;
- soumettre le composé ainsi immobilisé à des conditions permettant son clivage ; et

- récupérer le composé clivé purifié.

**13.** Biopuce ou biocapteur susceptible d'être préparé(e) en utilisant un support solide selon la revendication 7 et un composé selon l'une quelconque des revendications 8 à 10, ledit composé étant conjugué avec l'un des deux peptides tels que définis à la revendication 1 et l'autre peptide tel que défini à la revendication 1 étant immobilisé sur ledit support solide.

**14.** Composé selon l'une quelconque des revendications 8 à 10 pour utilisation en médecine ou pour le diagnostic.

**15.** Kit de diagnostic ou de traitement comprenant :

- dans un 1$^{er}$ compartiment, un 1$^{er}$ composé capable de reconnaître ou de cibler un site pathologique et conjugué avec un des deux peptides tel que défini à la revendication 1 ou un précurseur d'un tel composé ; et
- dans un 2$^{nd}$ compartiment, un 2$^{nd}$ composé facilement détectable ou cytotoxique marqué par l'autre peptide tel que défini à la revendication 1.

**16.** Procédé pour cristalliser un composé, comprenant les étapes consistant à :

- conjuguer ledit composé avec un des deux peptides tels que définis à la revendication 1 ;
- mettre le composé ainsi conjugué en présence de l'autre peptide tel que défini à la revendication 1 ;
- soumettre le composé ainsi obtenu à des conditions permettant sa cristallisation.

**17.** Structure circulaire comprenant au moins un composé tel que défini à l'une quelconque des revendications 8 à 10.

**18.** Structure multimérique comprenant au moins deux composés tels que définis à l'une quelconque des revendications 8 à 10 et, avantageusement une pluralité de composés tels que définis à l'une quelconque des revendications 8 à 10, identiques ou différents, chaque composé étant lié à au moins un autre composé par une liaison isopeptidique.

**19.** Utilisation d'une structure circulaire selon la revendication 17 ou d'une structure multimérique selon la revendication 18 pour la préparation d'anticorps.

**Patentansprüche**

**1.** Peptid zum Bilden eines kovalenten Komplexes, bestehend aus:

- entweder das Peptid, genannt Peptid Jo, dessen Aminosäuresequenz die folgende ist:

QYPQTGTYPDVQTPYQIIKVDGSEKNGQHKALNPNPYERVIPEGTLSK    RIYQVNNLDDNQYGIELTVSGKTVYEQ (SEQ ID NO: 2 in der beigefügten Sequenzliste), ein Derivat oder ein Fragment davon;

- oder das Peptid, genannt Peptid In, dessen Aminosäuresequenz die folgende ist:

IENGTITDPMGELIDLQLGTDGRFDPADYTLTANDGSRLENGQAVGGP QNDGGLLKNAKVLYDTTEKRIRVTGLYLGTDEKVTLTYNVRLNDEFVSNKFYDTN        GRTTLHPKEVEQNTVRDFPIPKIRDVR (SEQ ID NO: 4 in der beigefügten Sequenzliste), ein Derivat oder ein Fragment davon,
wobei die Derivate und Fragmente dazu ausgelegt sind, spontan einen kovalenten Komplex mit Hilfe einer Isopeptidverbindung zu bilden,
wobei ein Derivat des Peptids Jo und ein Derivat des Peptids In wenigstens 40% Identität mit SEQ ID NO: 2 beziehungsweise mit SEQ ID NO: 4 aufweisen.

**2.** Heterodimer, gebildet durch die zwei Peptide wie in Anspruch 1 definiert, die durch eine Isopeptidverbindung miteinander verbunden sind.

**3.** Isoliertes Polynukleotid, das verschieden ist von dem natürlichen Polynukleotid, das das Protein RrgA von *Streptococcus pneumoniae* codiert, ausgewählt aus den nachfolgenden unterschiedlichen Polynukleotiden:

i) ein Polynukleotid, das ein Peptid wie in Anspruch 1 definiert codiert;

ii) ein komplementäres Polynukleotid des Polynukleotids wie in Punkt (i) definiert;

iii) ein Polynukleotid von wenigstens 18 Nukleotiden, das dazu ausgelegt ist, sich unter Bedingungen starker Stringenz zu den Polynukleotiden wie in den Punkten (i) und (ii) definiert zu hybridisieren,

wobei das Polynukleotid (iii), das dazu ausgelegt ist, sich unter Bedingungen starker Stringenz mit dem Polynukleotid wie in Punkt (ii) definiert zu hybridisieren, ein Peptid codiert, das dazu ausgelegt ist, spontan einen kovalenten Komplex mit Hilfe einer Isopeptidverbindung zu bilden.

4. Polynukleotid nach Anspruch 3, **dadurch gekennzeichnet, dass** es wenigstens eine Nukleotidsequenz umfasst, die wenigstens 40%, wenigstens 45%, wenigstens 50%, wenigstens 55%, wenigstens 60%, wenigstens 65%, wenigstens 70%, wenigstens 75%, wenigstens 80%, wenigstens 85%, wenigstens 90%, wenigstens 95% und/oder wenigstens 99% Identität mit einer der nachfolgenden Nukleotidsequenzen oder einer ihrer komplementären Sequenzen aufweist:

CAGTATCCACAAACAGGGACTTATCCAGATGTTCAAACACCTTATC AGATTATTAAGGTAGATGGTT-CGGAAAAAAACGGACAGCACAAGGCGTTGAA TCCGAATCCATATGAACGTGTGATTCCAGAAGGTA-CACTTTCAAAGAGAATTT ATCAAGTGAATAATTTGGATGATAACCAATATGGAATCGAATTGACGGT-TAGT GGGAAAACAGTGTATGAACAA (SEQ ID NO: 1 der beigefügten Sequenzliste), oder

ATTGAGAATGGTACGATTACAGATCCGATGGGTGAGTTAATTGATT
TGCAATTGGGCACAGATGGAAGATTTGATCCAGCAGATTACACTTTAACTGCA
AACGATGGTAGTCGCTTGGAGAATGGACAAGCTGTAGGTGGTCCACAAAATG
ATGGTGGTTTGTTAAAAAATGCAAAAGTGCTCTATGATACGACTGAGAAAAGG
ATTCGTGTAACAGGTCTGTACCTTGGAACGGATGAAAAAGTTACGTTGACCTA
CAATGTTCGTTTGAATGATGAGTTTGTAAGCAATAAATTTTATGATACCAATGG
TCGAACAACCTTACATCCTAAGGAAGTAGAACAGAACACAGTGCGCGACTTC CCGATTCCTAAGATTCGTGAT (SEQ ID NO: 3 der beigefügten Sequenzliste),

wobei die Nukleotidsequenz ein Peptid codiert, das dazu ausgelegt ist, spontan einen kovalenten Komplex mit Hilfe einer Isopeptidverbindung zu bilden.

5. Klonierungs- und/oder Expressionsvektor, umfassend wenigstens ein Polynukleotid wie in Anspruch 3 oder 4 definiert.

6. Wirtsorganismus, transformiert durch oder umfassend ein Polynukleotid wie in Anspruch 3 oder 4 definiert, oder einen Vektor wie in Anspruch 5 definiert.

7. Fester Träger, an dessen Oberfläche ein (oder mehrere) Peptid(e) wie in Anspruch 1 definiert immobilisiert ist/sind.

8. Konjugierte Verbindung mit wenigstens einem Peptid wie in Anspruch 1 definiert.

9. Verbindung nach Anspruch 8, **dadurch gekennzeichnet, dass** die Verbindung ein biologisches oder biologisch aktives Molekül, ein Monomer, eine leicht detektierbare Verbindung oder eine zytotoxische Verbindung ist.

10. Verbindung nach Anspruch 8 oder 9, **dadurch gekennzeichnet, dass** die Verbindung durch zwei identische oder unterschiedliche Peptide wie in Anspruch 1 definiert konjugiert ist.

11. Verwendung eines festen Trägers nach Anspruch 7 und einer Verbindung nach einem der Ansprüche 8 bis 10 zum Reinigen der Verbindung oder zum Herstellen eines Biochips oder eines Biosensors, wobei die Verbindung mit einem der zwei Peptide wie in Anspruch 1 definiert konjugiert ist, und das andere Peptid wie in Anspruch 1 definiert auf dem festen Träger immobilisiert ist.

12. Verfahren zum Reinigen einer Verbindung, umfassend die folgenden Schritte:

- indirektes Konjugieren der Verbindung mit einem der zwei Peptide wie in Anspruch 1 definiert, wobei ein spaltbarer Abstandhalterarm die Verbindung und das Peptid trennt;
- Herstellen eines festen Trägers, an dessen Oberfläche das andere Peptid wie in Anspruch 1 definiert immobilisiert ist;
- Verbringen der derart konjugierten Verbindung in Anwesenheit des derart hergestellten Trägers, wodurch die konjugierte Verbindung auf dem festen Träger immobilisiert wird;
- Aussetzen der derart immobilisierten Verbindung an Umstände, die ihre Spaltung erlauben; und
- Aufsammeln der gereinigten gespaltenen Verbindung.

**13.** Biochip oder Biosensor, der unter Verwendung eines festen Trägers nach Anspruch 7 und einer Verbindung nach einem der Ansprüche 8 bis 10 herstellbar ist,
wobei die Verbindung mit einem der zwei Peptide wie in Anspruch 1 definiert konjugiert ist, und das andere Peptid wie in Anspruch 1 definiert auf dem festen Träger immobilisiert ist.

**14.** Verbindung nach einem der Ansprüche 8 bis 10 zur Verwendung in der Medizin oder für die Diagnostik.

**15.** Diagnostik- oder Behandlungsset, umfassend:

- in einem ersten Fach, eine erste Verbindung, die dazu ausgelegt ist, eine pathologische Stelle zu erkennen oder anzuvisieren, und die mit einem der zwei Peptide wie in Anspruch 1 definiert konjugiert ist, oder einen Prekursor einer derartigen Verbindung; und
- in einem zweiten Fach, eine leicht detektierbare oder zytotoxische zweite Verbindung, die durch das andere Peptid wie in Anspruch 1 definiert markiert ist.

**16.** Verfahren zum Kristallisieren einer Verbindung, umfassend die folgenden Schritte:

- Konjugieren der Verbindung mit einem der zwei Peptide wie in Anspruch 1 definiert;
- Verbringen der derart konjugierten Verbindung in Anwesenheit des anderen Peptids wie in Anspruch 1 definiert;
- Aussetzen der derart erhaltenen Verbindung an Bedingungen, die ihre Kristallisation erlauben.

**17.** Kreisstruktur, umfassend wenigstens eine Verbindung wie in einem der Ansprüche 8 bis 10 definiert.

**18.** Multimerstruktur, umfassend wenigstens zwei Verbindungen wie in einem der Ansprüche 8 bis 10 definiert, und vorzugsweise eine Mehrzahl von Verbindungen wie in einem der Ansprüche 8 bis 10 definiert, die identisch oder unterschiedlich sind, wobei jede Verbindung mit wenigstens einer anderen Verbindung durch eine Isopeptidverbindung verbunden ist.

**19.** Verwendung einer Kreisstruktur nach Anspruch 17 oder einer Multimerstruktur nach Anspruch 18 für die Herstellung von Antikörpern.

**Claims**

**1.** Peptide capable of forming a covalent complex consisting in:

- either the peptide, designated Jo peptide, the amino acid sequence of which is the following:

    QYPQTGTYPDVQTPYQIIKVDGSEKNGQHKALNPNPYERVIPEGTLSKRIYQVNNLDDN QYGIELTVSGKTVYEQ (SEQ ID NO: 2 in the appended sequence listing), a derivative or a fragment thereof;

- or the peptide, designated In peptide, the amino acid sequence of which is the following:

    IENGTITDPMGELIDLQLGTDGRFDPADYTLTANDGSRLENGQAVGGPQNDGGLLKNA KVLYDTTEKRIRVTGLYLGTDEKVTLTYNVRLNDEFVSNKFYDTNGRTTLHPKEVEQNTVRDFPIPKIRD VR (SEQ ID NO: 4 in the appended sequence listing), a derivative or a fragment thereof,
    said derivatives and fragments being capable of spontaneously forming a covalent complex by means of an isopeptide bond,
    a derivative of the Jo peptide and a derivative of the In peptide having at least 40% of identity respectively with SEQ ID NO: 2 and SEQ ID NO: 4.

**2.** Heterodimer formed by the two peptides as defined in claim 1 and bound to each other by an isopeptide bond.

**3.** Isolated polynucleotide, different from the natural polynucleotide encoding the RrgA protein of *Streptococcus pneu-*

*moniae,* selected from the different polynucleotides hereafter:

i) a polynucleotide encoding a peptide as defined in claim 1;
ii) a complementary polynucleotide of the polynucleotide as defined in point (i);
iii) a polynucleotide of at least 18 nucleotides, capable of hybridizing itself in conditions of high stringency to polynucleotides as defined in points (i) and (ii),
said polynucleotide (iii) capable of hybridizing itself in conditions of high stringency to polynucleotide as defined in point (ii) encodes a peptide capable of spontaneously forming a covalent complex by means of an isopeptide bond.

4. Polynucleotide according to claim 3, **characterised in that** it comprises at least one nucleotide sequence having at least 40%, at least 45%, at least 50%, at least 55%, at least 60%, at least 65%, at least 70%, at least 75%, at least 80%, at least 85%, at least 90%, at least 95% and/or at least 99% of identity with one of the nucleotide sequences hereafter or one of the complementary sequences thereof:

CAGTATCCACAAACAGGGACTTATCCAGATGTTCAAACACCTTATCAGATTATTAAG
GTAGATGGTTCGGAAAAAAACGGACAGCACAAGGCGTTGAATCCGAATCCATATGAACGTGTGA
TTCCAGAAGGTACACTTTCAAAGAGAATTTATCAAGTGAATAATTTGGATGATAACCAATATGGA
ATCGAATTGACGGTTAGTGGGAAAACAGTGTATGAACAA (SEQ ID NO: 1 in the appended listing of sequences) or
ATTGAGAATGGTACGATTACAGATCCGATGGGTGAGTTAATTGATTTGCAATTGGG
CACAGATGGAAGATTTGATCCAGCAGATTACACTTTAACTGCAAACGATGGTAGTCGCTTGGAGA
ATGGACAAGCTGTAGGTGGTCCACAAAATGATGGTGGTTTGTTAAAAAATGCAAAAGTGCTCTAT
GATACGACTGAGAAAAGGATTCGTGTAACAGGTCTGTACCTTGGAACGGATGAAAAAGTTACGT
TGACCTACAATGTTCGTTTGAATGATGAGTTTGTAAGCAATAAATTTTATGATACCAATGGTCGAA
CAACCTTACATCCTAAGGAAGTAGAACAGAACACAGTGCGCGACTTCCCGATTCCTAAGATTCGT    GAT
(SEQ ID NO: 3 in the appended listing of sequences),
said nucleotide sequence encodes a peptide capable of spontaneously forming a covalent complex by means of an isopeptide bond.

5. Cloning and/or expression vector containing at least one polynucleotide as defined in claim 3 or 4.

6. Host organism transformed by or comprising a polynucleotide as defined in claim 3 or 4 or a vector as defined in claim 5.

7. Solid support on the surface of which one (or more) peptide(s) as defined in claim 1 is(are) immobilized.

8. Compound conjugated with at least one peptide as defined in claim 1.

9. Compound according to claim 8, **characterised in that** said compound is a biological or biologically active molecule, a monomer, an easily detectable compound or a cytotoxic compound.

10. Compound according to claim 8 or 9, **characterised in that** said compound is conjugated by two peptides, identical or different, as defined in claim 1.

11. Use of a solid support according to claim 7 and of a compound according to any of claims 8 to 10, for purifying said compound or for preparing a biochip or a biosensor,
said compound being conjugated with one of the two peptides as defined in claim 1 and the other peptide as defined in claim 1 being immobilized on said solid support.

12. Method for purifying a compound, comprising the steps consisting in:

- conjugating indirectly said compound with one of the two peptides as defined in claim 1, a cleavable spacer arm separating said compound and said peptide;
- preparing a solid support on the surface of which the other peptide as defined in claim 1 is immobilized;
- placing the compound thereby conjugated in the presence of the support thereby prepared whereby the conjugated compound is immobilized on the solid support;
- subjecting the compound thereby immobilized to conditions enabling the cleavage thereof; and

- recovering the purified cleaved compound.

13. Biochip or biosensor capable of being prepared using a solid support according to claim 7 and a compound according to any of claims 8 to 10,
   said compound being conjugated with one of the two peptides as defined in claim 1 and the other peptide as defined in claim 1 being immobilized on said solid support.

14. Compound according to any of claims 8 to 10 for use in medicine or for diagnostics.

15. Diagnostic or treatment kit comprising:

   - in a 1st compartment, a 1st compound capable of recognizing or targeting a pathologic site and conjugated with one of the two peptides as defined in claim 1 or a precursor of such a compound; and
   - in a 2nd compartment, a 2nd easily detectable or cytotoxic compound marked by the other peptide as defined in claim 1.

16. Method for crystallizing a compound, comprising the steps consisting in:

   - conjugating said compound with one of the two peptides as defined in claim 1;
   - placing the compound thereby conjugated in the presence of the other peptide as defined in claim 1;
   - subjecting the compound thereby obtained to conditions enabling the crystallization thereof.

17. Circular structure comprising at least one compound as defined in any of claims 8 to 10.

18. Multimeric structure comprising at least two compounds as defined in any of claims 8 to 10 and, advantageously, a plurality of compounds as defined in any of claims 8 to 10, identical or different, each compound being bound to at least one other compound by an isopeptide bond.

19. Use of a circular structure according to claim 17 or of a multimeric structure according to claim 18 for preparing antibodies.

FIG.1A

FIG.1B

FIG.1C

FIG.1D

FIG.2

Protéine modulaire

ou

Protéine modulaire

FIG.3A

FIG.3B

FIG.4

| IPTG | + | + | + | + | + | - | + |
|------|---|---|---|---|---|---|---|
| hisJo | + | + | - | - | + | - | + |
| In | - | - | + | + | + | - | + |

hisJoIn
25 826 Da

In
15 135 Da

hisJo
10 471 Da

## FIG.5

load  flow  E1  E2  E3    E4  PM

—250kDa
—150kDa
—100kDa
—75kDa

—50kDa

—37kDa

—hisJoIn
—25kDa

—20kDa

—hisJo
—15kDa

## FIG.6

56

HisJoHisIn

| | | 5 | HisJoIn | 1 | 2,5 | 5 | 5 | 5 | 5 |
| 5 | | | | 5 | 5 | 5 | 1 | 2,5 | 5 |

— JoIn

**FIG.7**

50kDa —
37kDa —
25kDa —

— FusJoIn
25 874 kDa

**FIG.8**

| | WT | WT | D600A | D600A | N695A | N695A | | WT | HisIn |
| WT | | WT | | WT | | WT | K191A | K191A | HisJo |

25kDa —
20kDa —
15kDa —

— HisJoHisIn
— HisIn
— HisJo

**FIG.9**

FIG.10

FIG.11

Dimère ⟶

Masse apparente 58kDa

⟵ Monomère

Masse apparente 32kDa

FIG.12

FIG.13

FIG.14

## RÉFÉRENCES CITÉES DANS LA DESCRIPTION

**Documents brevets cités dans la description**

- EP 2012XXXXXX W

- FR 1152432

**Littérature non-brevet citée dans la description**

- **ZAKERI ; HOWARTH.** *J. Am. Chem. Soc.,* 2010, vol. 132, 4526-4527
- **IZORÉ et al.** *Structure,* 2010, vol. 18, 106-115
- **SAMBROOK et al.** Molecular cloning. Cold Spring Harbor Laboratory Press, 1989

- **MILLER ; LONG.** *Science,* 2012, vol. 335, 432-436
- **BROHAWN et al.** *Science,* 2012, vol. 335, 436-441

**SEQUENCE LISTING**

<110> Commissariat à l'Energie Atomique et aux Energies Alternatives (CEA)

<120> PEPTIDES APTES A FORMER UN COMPLEXE COVALENT ET LEURS UTILISATIONS

<130> SP39394 (PCT)/SG

<140> PCT/EP2012/XXXXXX
<141> 2012-03-26

<150> FR11/52432
<151> 2011-03-24

<160> 22

<170> PatentIn version 3.5

<210> 1
<211> 225
<212> DNA
<213> Streptococcus pneumoniae

<220>
<221> CDS
<222> (1)..(225)
<223> Séquence codant le peptide Jo

<400> 1

```
cag tat cca caa aca ggg act tat cca gat gtt caa aca cct tat cag        48
Gln Tyr Pro Gln Thr Gly Thr Tyr Pro Asp Val Gln Thr Pro Tyr Gln
1               5                   10                  15

att att aag gta gat ggt tcg gaa aaa aac gga cag cac aag gcg ttg        96
Ile Ile Lys Val Asp Gly Ser Glu Lys Asn Gly Gln His Lys Ala Leu
                20                  25                  30

aat ccg aat cca tat gaa cgt gtg att cca gaa ggt aca ctt tca aag       144
Asn Pro Asn Pro Tyr Glu Arg Val Ile Pro Glu Gly Thr Leu Ser Lys
            35                  40                  45

aga att tat caa gtg aat aat ttg gat gat aac caa tat gga atc gaa       192
Arg Ile Tyr Gln Val Asn Asn Leu Asp Asp Asn Gln Tyr Gly Ile Glu
        50                  55                  60

ttg acg gtt agt ggg aaa aca gtg tat gaa caa                           225
Leu Thr Val Ser Gly Lys Thr Val Tyr Glu Gln
65                  70                  75
```

<210> 2
<211> 75
<212> PRT
<213> Streptococcus pneumoniae

<400> 2

```
Gln Tyr Pro Gln Thr Gly Thr Tyr Pro Asp Val Gln Thr Pro Tyr Gln
1               5                   10                  15

Ile Ile Lys Val Asp Gly Ser Glu Lys Asn Gly Gln His Lys Ala Leu
                20                  25                  30

Asn Pro Asn Pro Tyr Glu Arg Val Ile Pro Glu Gly Thr Leu Ser Lys
            35                  40                  45

Arg Ile Tyr Gln Val Asn Asn Leu Asp Asp Asn Gln Tyr Gly Ile Glu
        50                  55                  60

Leu Thr Val Ser Gly Lys Thr Val Tyr Glu Gln
65                  70                  75
```

<210> 3
<211> 390
<212> DNA
<213> Streptococcus pneumoniae

<220>
<221> CDS
<222> (1)..(384)
<223> Séquence codant le peptide In

<400> 3

```
att gag aat ggt acg att aca gat ccg atg ggt gag tta att gat ttg      48
Ile Glu Asn Gly Thr Ile Thr Asp Pro Met Gly Glu Leu Ile Asp Leu
1               5               10              15

caa ttg ggc aca gat gga aga ttt gat cca gca gat tac act tta act      96
Gln Leu Gly Thr Asp Gly Arg Phe Asp Pro Ala Asp Tyr Thr Leu Thr
            20              25              30

gca aac gat ggt agt cgc ttg gag aat gga caa gct gta ggt ggt cca     144
Ala Asn Asp Gly Ser Arg Leu Glu Asn Gly Gln Ala Val Gly Gly Pro
        35              40              45

caa aat gat ggt ggt ttg tta aaa aat gca aaa gtg ctc tat gat acg     192
Gln Asn Asp Gly Gly Leu Leu Lys Asn Ala Lys Val Leu Tyr Asp Thr
    50              55              60

act gag aaa agg att cgt gta aca ggt ctg tac ctt gga acg gat gaa     240
Thr Glu Lys Arg Ile Arg Val Thr Gly Leu Tyr Leu Gly Thr Asp Glu
65              70              75              80

aaa gtt acg ttg acc tac aat gtt cgt ttg aat gat gag ttt gta agc     288
Lys Val Thr Leu Thr Tyr Asn Val Arg Leu Asn Asp Glu Phe Val Ser
            85              90              95

aat aaa ttt tat gat acc aat ggt cga aca acc tta cat cct aag gaa     336
Asn Lys Phe Tyr Asp Thr Asn Gly Arg Thr Thr Leu His Pro Lys Glu
            100             105             110

gta gaa cag aac aca gtg cgc gac ttc ccg att cct aag att cgt gat     384
Val Glu Gln Asn Thr Val Arg Asp Phe Pro Ile Pro Lys Ile Arg Asp
        115             120             125

cgtgat                                                              390
```

<210> 4
<211> 128
<212> PRT
<213> Streptococcus pneumoniae

<400> 4

```
Ile Glu Asn Gly Thr Ile Thr Asp Pro Met Gly Glu Leu Ile Asp Leu
1               5                   10                  15


Gln Leu Gly Thr Asp Gly Arg Phe Asp Pro Ala Asp Tyr Thr Leu Thr
            20                  25                  30


Ala Asn Asp Gly Ser Arg Leu Glu Asn Gly Gln Ala Val Gly Gly Pro
            35                  40                  45


Gln Asn Asp Gly Gly Leu Leu Lys Asn Ala Lys Val Leu Tyr Asp Thr
    50                  55                  60


Thr Glu Lys Arg Ile Arg Val Thr Gly Leu Tyr Leu Gly Thr Asp Glu
65                  70                  75                  80


Lys Val Thr Leu Thr Tyr Asn Val Arg Leu Asn Asp Glu Phe Val Ser
                85                  90                  95


Asn Lys Phe Tyr Asp Thr Asn Gly Arg Thr Thr Leu His Pro Lys Glu
            100                 105                 110


Val Glu Gln Asn Thr Val Arg Asp Phe Pro Ile Pro Lys Ile Arg Asp
            115                 120                 125
```

<210> 5
<211> 237
<212> DNA
<213> Unknown

<220>
<223> Artificial sequence

<220>
<221> CDS
<222> (1)..(237)
<223> Séquence codant un dérivé du peptide Jo

<400> 5

```
tct gac cag tat cca caa aca ggg act tat cca gat gtt caa aca cct       48
Ser Asp Gln Tyr Pro Gln Thr Gly Thr Tyr Pro Asp Val Gln Thr Pro
1               5                   10                  15


tat cag att att aag gta gat ggt tcg gaa aaa aac gga cag cac aag       96
Tyr Gln Ile Ile Lys Val Asp Gly Ser Glu Lys Asn Gly Gln His Lys
                20                  25                  30
```

```
gcg ttg aat ccg aat cca tat gaa cgt gtg att cca gaa ggt aca ctt      144
Ala Leu Asn Pro Asn Pro Tyr Glu Arg Val Ile Pro Glu Gly Thr Leu
        35              40                  45

tca aag aga att tat caa gtg aat aat ttg gat gat aac caa tat gga      192
Ser Lys Arg Ile Tyr Gln Val Asn Asn Leu Asp Asp Asn Gln Tyr Gly
    50              55                  60

atc gaa ttg acg gtt agt ggg aaa aca gtg tat gaa caa aaa gat          237
Ile Glu Leu Thr Val Ser Gly Lys Thr Val Tyr Glu Gln Lys Asp
65                  70                  75
```

<210> 6
<211> 79
<212> PRT
<213> Unknown

<220>
<223> Synthetic Construct

<400> 6

```
Ser Asp Gln Tyr Pro Gln Thr Gly Thr Tyr Pro Asp Val Gln Thr Pro
1               5                   10                  15

Tyr Gln Ile Ile Lys Val Asp Gly Ser Glu Lys Asn Gly Gln His Lys
            20                  25                  30

Ala Leu Asn Pro Asn Pro Tyr Glu Arg Val Ile Pro Glu Gly Thr Leu
            35                  40                  45

Ser Lys Arg Ile Tyr Gln Val Asn Asn Leu Asp Asp Asn Gln Tyr Gly
        50                  55                  60

Ile Glu Leu Thr Val Ser Gly Lys Thr Val Tyr Glu Gln Lys Asp
65                  70                  75
```

<210> 7
<211> 399
<212> DNA
<213> Unknown

<220>
<223> Artificial sequence

<220>
<221> CDS
<222> (1)..(399)
<223> Séquence codant un dérivé du peptide In

<400> 7

```
act gaa aag aaa tca att gag aat ggt acg att aca gat ccg atg ggt          48
Thr Glu Lys Lys Ser Ile Glu Asn Gly Thr Ile Thr Asp Pro Met Gly
1               5                   10                  15

gag tta att gat ttg caa ttg ggc aca gat gga aga ttt gat cca gca          96
Glu Leu Ile Asp Leu Gln Leu Gly Thr Asp Gly Arg Phe Asp Pro Ala
                20                  25                  30

gat tac act tta act gca aac gat ggt agt cgc ttg gag aat gga caa         144
Asp Tyr Thr Leu Thr Ala Asn Asp Gly Ser Arg Leu Glu Asn Gly Gln
            35                  40                  45

gct gta ggt ggt cca caa aat gat ggt ggt ttg tta aaa aat gca aaa         192
Ala Val Gly Gly Pro Gln Asn Asp Gly Gly Leu Leu Lys Asn Ala Lys
        50                  55                  60

gtg ctc tat gat acg act gag aaa agg att cgt gta aca ggt ctg tac         240
Val Leu Tyr Asp Thr Thr Glu Lys Arg Ile Arg Val Thr Gly Leu Tyr
65                  70                  75                  80

ctt gga acg gat gaa aaa gtt acg ttg acc tac aat gtt cgt ttg aat         288
Leu Gly Thr Asp Glu Lys Val Thr Leu Thr Tyr Asn Val Arg Leu Asn
                85                  90                  95

gat gag ttt gta agc aat aaa ttt tat gat acc aat ggt cga aca acc         336
Asp Glu Phe Val Ser Asn Lys Phe Tyr Asp Thr Asn Gly Arg Thr Thr
                100                 105                 110

tta cat cct aag gaa gta gaa cag aac aca gtg cgc gac ttc ccg att         384
Leu His Pro Lys Glu Val Glu Gln Asn Thr Val Arg Asp Phe Pro Ile
            115                 120                 125

cct aag att cgt gat                                                     399
Pro Lys Ile Arg Asp
            130
```

<210> 8
<211> 133
<212> PRT
<213> Unknown

<220>
<223> Synthetic Construct

<400> 8

```
Thr Glu Lys Lys Ser Ile Glu Asn Gly Thr Ile Thr Asp Pro Met Gly
1               5                   10                  15

Glu Leu Ile Asp Leu Gln Leu Gly Thr Asp Gly Arg Phe Asp Pro Ala
            20                  25                  30

Asp Tyr Thr Leu Thr Ala Asn Asp Gly Ser Arg Leu Glu Asn Gly Gln
        35                  40                  45

Ala Val Gly Gly Pro Gln Asn Asp Gly Gly Leu Leu Lys Asn Ala Lys
        50                  55                  60

Val Leu Tyr Asp Thr Thr Glu Lys Arg Ile Arg Val Thr Gly Leu Tyr
65                  70                  75                  80

Leu Gly Thr Asp Glu Lys Val Thr Leu Thr Tyr Asn Val Arg Leu Asn
                85                  90                  95

Asp Glu Phe Val Ser Asn Lys Phe Tyr Asp Thr Asn Gly Arg Thr Thr
            100                 105                 110

Leu His Pro Lys Glu Val Glu Gln Asn Thr Val Arg Asp Phe Pro Ile
            115                 120                 125

Pro Lys Ile Arg Asp
        130
```

<210> 9
<211> 92
<212> PRT
<213> Unknown

<220>
<223> Artificial sequence

<220>
<221> PEPTIDE
<222> (1)..(92)
<223> Peptide HisJo

<400> 9

```
Gly Ser Ser His His His His His His Ser Gln Asp Pro Ser Asp Gln
1               5                   10              15

Tyr Pro Gln Thr Gly Thr Tyr Pro Asp Val Gln Thr Pro Tyr Gln Ile
            20              25              30

Ile Lys Val Asp Gly Ser Glu Lys Asn Gly Gln His Lys Ala Leu Asn
            35              40              45

Pro Asn Pro Tyr Glu Arg Val Ile Pro Glu Gly Thr Leu Ser Lys Arg
        50              55              60

Ile Tyr Gln Val Asn Asn Leu Asp Asp Asn Gln Tyr Gly Ile Glu Leu
65              70              75              80

Thr Val Ser Gly Lys Thr Val Tyr Glu Gln Lys Asp
                85              90
```

<210> 10
<211> 146
<212> PRT
<213> Unknown

<220>
<223> Artificial sequence

<220>
<221> PEPTIDE
<222> (1)..(146)
<223> Peptide HisIn

<400> 10

Gly Ser Ser His His His His His Ser Gln Asp Pro Thr Glu Lys
1                   5                   10                  15

Lys Ser Ile Glu Asn Gly Thr Ile Thr Asp Pro Met Gly Glu Leu Ile
            20                  25                  30

Asp Leu Gln Leu Gly Thr Asp Gly Arg Phe Asp Pro Ala Asp Tyr Thr
            35                  40                  45

Leu Thr Ala Asn Asp Gly Ser Arg Leu Glu Asn Gly Gln Ala Val Gly
        50                  55                  60

Gly Pro Gln Asn Asp Gly Gly Leu Leu Lys Asn Ala Lys Val Leu Tyr
65                  70                  75                  80

Asp Thr Thr Glu Lys Arg Ile Arg Val Thr Gly Leu Tyr Leu Gly Thr
                85                  90                  95

Asp Glu Lys Val Thr Leu Thr Tyr Asn Val Arg Leu Asn Asp Glu Phe
            100                 105                 110

Val Ser Asn Lys Phe Tyr Asp Thr Asn Gly Arg Thr Thr Leu His Pro
            115                 120                 125

Lys Glu Val Glu Gln Asn Thr Val Arg Asp Phe Pro Ile Pro Lys Ile
    130                 135                 140

Arg Asp
145

<210> 11
<211> 228
<212> PRT
<213> Unknown

<220>
<223> Artificial sequence

<220>
<221> PEPTIDE
<222> (1)..(228)
<223> Peptide HisJoIn

<400> 11

```
Gly Ser Ser His His His His His His Ser Gln Asp Pro Ser Asp Gln
1               5                   10              15

Tyr Pro Gln Thr Gly Thr Tyr Pro Asp Val Gln Thr Pro Tyr Gln Ile
        20                  25              30

Ile Lys Val Asp Gly Ser Glu Lys Asn Gly Gln His Lys Ala Leu Asn
        35              40              45

Pro Asn Pro Tyr Glu Arg Val Ile Pro Glu Gly Thr Leu Ser Lys Arg
        50              55              60

Ile Tyr Gln Val Asn Asn Leu Asp Asp Asn Gln Tyr Gly Ile Glu Leu
65              70              75              80

Thr Val Ser Gly Lys Thr Val Tyr Glu Gln Lys Asp Ala Asp Leu Thr
                85              90              95

Glu Lys Lys Ser Ile Glu Asn Gly Thr Ile Thr Asp Pro Met Gly Glu
                100             105             110

Leu Ile Asp Leu Gln Leu Gly Thr Asp Gly Arg Phe Asp Pro Ala Asp
        115             120             125

Tyr Thr Leu Thr Ala Asn Asp Gly Ser Arg Leu Glu Asn Gly Gln Ala
    130             135             140

Val Gly Gly Pro Gln Asn Asp Gly Gly Leu Leu Lys Asn Ala Lys Val
145             150             155             160

Leu Tyr Asp Thr Thr Glu Lys Arg Ile Arg Val Thr Gly Leu Tyr Leu
                165             170             175

Gly Thr Asp Glu Lys Val Thr Leu Thr Tyr Asn Val Arg Leu Asn Asp
        180             185             190

Glu Phe Val Ser Asn Lys Phe Tyr Asp Thr Asn Gly Arg Thr Thr Leu
        195             200             205

His Pro Lys Glu Val Glu Gln Asn Thr Val Arg Asp Phe Pro Ile Pro
    210             215             220

Lys Ile Arg Asp
225
```

<210> 12
<211> 239

<212> PRT
<213> Unknown

<220>
<223> Artificial sequence

<220>
<221> PEPTIDE
<222> (1)..(239)
<223> Peptide HisJoHisIn

<400> 12

```
Gly Ser Ser His His His His His Ser Gln Asp Pro Ser Asp Gln
1               5               10              15

Tyr Pro Gln Thr Gly Thr Tyr Pro Asp Val Gln Thr Pro Tyr Gln Ile
        20              25              30

Ile Lys Val Asp Gly Ser Glu Lys Asn Gly Gln His Lys Ala Leu Asn
        35              40              45

Pro Asn Pro Tyr Glu Arg Val Ile Pro Glu Gly Thr Leu Ser Lys Arg
    50              55              60

Ile Tyr Gln Val Asn Asn Leu Asp Asp Asn Gln Tyr Gly Ile Glu Leu
65              70              75              80

Thr Val Ser Gly Lys Thr Val Tyr Glu Gln Lys Asp Met Gly Ser Ser
                85              90              95

His His His His His His Ser Gln Asp Pro Thr Glu Lys Lys Ser Ile
        100             105             110

Glu Asn Gly Thr Ile Thr Asp Pro Met Gly Glu Leu Ile Asp Leu Gln
        115             120             125

Leu Gly Thr Asp Gly Arg Phe Asp Pro Ala Asp Tyr Thr Leu Thr Ala
    130             135             140

Asn Asp Gly Ser Arg Leu Glu Asn Gly Gln Ala Val Gly Gly Pro Gln
145             150             155             160

Asn Asp Gly Gly Leu Leu Lys Asn Ala Lys Val Leu Tyr Asp Thr Thr
                165             170             175

Glu Lys Arg Ile Arg Val Thr Gly Leu Tyr Leu Gly Thr Asp Glu Lys
            180             185             190

Val Thr Leu Thr Tyr Asn Val Arg Leu Asn Asp Glu Phe Val Ser Asn


        195             200             205

Lys Phe Tyr Asp Thr Asn Gly Arg Thr Thr Leu His Pro Lys Glu Val
    210             215             220

Glu Gln Asn Thr Val Arg Asp Phe Pro Ile Pro Lys Ile Arg Asp
225             230             235
```

<210> 13

EP 2 688 905 B1

<211> 232
<212> PRT
<213> Unknown

<220>
<223> Artificial sequence

<220>
<221> PEPTIDE
<222> (1)..(232)
<223> Peptide FusJoln

<400> 13

```
Gly Ser Ser His His His His His His Ser Gln Asp Pro Ser Asp Gln
1               5                   10                  15

Tyr Pro Gln Thr Gly Thr Tyr Pro Asp Val Gln Thr Pro Tyr Gln Ile
            20                  25                  30

Ile Lys Val Asp Gly Ser Glu Lys Asn Gly Gln His Lys Ala Leu Asn
            35                  40                  45

Pro Asn Pro Tyr Glu Arg Val Ile Pro Glu Gly Thr Leu Ser Lys Arg
        50                  55                  60

Ile Tyr Gln Val Asn Asn Leu Asp Asp Asn Gln Tyr Gly Ile Glu Leu
65                  70                  75                  80

Thr Val Ser Gly Lys Thr Val Tyr Glu Gln Lys Asp Gly Ser Thr Pro
                85                  90                  95

Gly Ser Val Thr Glu Lys Lys Ser Ile Glu Asn Gly Thr Ile Thr Asp
            100                 105                 110

Pro Met Gly Glu Leu Ile Asp Leu Gln Leu Gly Thr Asp Gly Arg Phe
            115                 120                 125

Asp Pro Ala Asp Tyr Thr Leu Thr Ala Asn Asp Gly Ser Arg Leu Glu
        130                 135                 140
```

37

```
        Asn Gly Gln Ala Val Gly Gly Pro Gln Asn Asp Gly Gly Leu Leu Lys
        145             150             155             160

        Asn Ala Lys Val Leu Tyr Asp Thr Thr Glu Lys Arg Ile Arg Val Thr
                        165             170             175

        Gly Leu Tyr Leu Gly Thr Asp Glu Lys Val Thr Leu Thr Tyr Asn Val
                        180             185             190

        Arg Leu Asn Asp Glu Phe Val Ser Asn Lys Phe Tyr Asp Thr Asn Gly
                        195             200             205

        Arg Thr Thr Leu His Pro Lys Glu Val Glu Gln Asn Thr Val Arg Asp
            210             215             220

        Phe Pro Ile Pro Lys Ile Arg Asp
        225             230
```

<210> 14
<211> 86
<212> DNA
<213> Unknown

<220>
<223> Séquence dans le plasmide dérivé de pET-Duet-1

<220>
<221> misc_feature
<222> (1)..(35)
<223> Séquence partielle d'un dérivé de Jo

<220>
<221> CDS
<222> (36)..(56)
<223> Séquence codant un linker de 7 acides aminés

<220>
<221> misc_feature
<222> (57)..(86)
<223> séquence partielle d'un dérivé de In

<400> 14

```
cggttagtgg gaaaacagtg tatgaacaaa aagat ggt tct acc ccg ggt tct        53
                                        Gly Ser Thr Pro Gly Ser
                                        1               5

gtt actgaaaaga aatcaattga gaatggtacg                                  86
Val
```

<210> 15
<211> 7
<212> PRT
<213> Unknown

<220>
<223> Synthetic Construct

<400> 15

```
                              Gly Ser Thr Pro Gly Ser Val
                              1                   5
```

<210> 16
<211> 98
<212> DNA
<213> Unknown

<220>
<223> Oligomère 1

<400> 16

```
cggttagtgg gaaaacagtg tatgaacaaa aagatggaat tcggtacccc tgcagggagc      60

tgaagcttac tgaaaagaaa tcaattgaga atggtacg                              98
```

<210> 17
<211> 98
<212> DNA
<213> Unknown

<220>
<223> Oligomère 2

<400> 17

```
cgtaccattc tcaattgatt tcttttcagt aagcttcagc tccctgcagg ggtaccgaat      60

tccatctttt tgttcataca ctgttttccc actaaccg                              98
```

<210> 18
<211> 30
<212> DNA
<213> Unknown

<220>
<223> Oligomère 3

<400> 18
gcgcgaattc cagaaatttc tcagcgtacg 30

<210> 19
<211> 27
<212> DNA
<213> Unknown

<220>
<223> Oligomère 4

<400> 19

gcgcaagctt gctgatttgg tttgatg 27

<210> 20
<211> 756
<212> DNA
<213> Unknown

<220>
<223> Séquence nucléotidique de la région pertinente du vecteur d clonage permettant l'expression d'une protéine de fusion ayant des dérivés de Jo et In respectivement en N- et C-terminus de la protéine

<220>
<221> misc_feature
<222> (43)..(60)
<223> Séquence nucléotidique codant pour 6 histidines

<220>
<221> misc_feature
<222> (73)..(309)
<223> Séquence nucléotidique codant pour un dérivé de Jo

<220>
<221> misc_feature
<222> (310)..(342)
<223> Site de clonage multiple (5 sites de restriction)

<220>
<221> misc_feature
<222> (311)..(316)
<223> Site de restriction EcoRI

<220>
<221> misc_feature
<222> (317)..(322)
<223> Site de restriction KpnI

<220>
<221> misc_feature
<222> (324)..(329)
<223> Site de restriction PstI

<220>
<221> misc_feature
<222> (331)..(336)
<223> Site de restriction SacI

<220>
<221> misc_feature
<222> (337)..(342)
<223> Site de restriction HindIII

<220>
<221> misc_feature
<222> (343)..(744)
<223> Séquence nucléotidique codant pour un dérivé de In

<400> 20

```
ttttgtttaa ctttaagaag gagatatacc atgggcagca gccatcacca tcatcaccac      60

agccaggatc cgtctgacca gtatccacaa acagggactt atccagatgt tcaaacacct     120

tatcagatta ttaaggtaga tggttcggaa aaaaacggac agcacaaggc gttgaatccg     180

aatccatatg aacgtgtgat tccagaaggt acactttcaa agagaattta tcaagtgaat     240

aatttggatg ataaccaata tggaatcgaa ttgacggtta gtgggaaaac agtgtatgaa     300

caaaaagatg gaattcggta cccctgcagg gagctgaagc ttactgaaaa gaaatcaatt     360

gagaatggta cgattacaga tccgatgggt gagttaattg atttgcaatt gggcacagat     420

ggaagatttg atccagcaga ttacacttta actgcaaacg atggtagtcg cttggagaat     480

ggacaagctg taggtggtcc acaaaatgat ggtggtttgt taaaaaatgc aaaagtgctc     540

tatgatacga ctgagaaaag gattcgtgta acaggtctgt accttggaac ggatgaaaaa     600

gttacgttga cctacaatgt tcgtttgaat gatgagtttg taagcaataa attttatgat     660

accaatggtc gaacaacctt acatcctaag gaagtagaac agaacacagt gcgcgacttc     720

ccgattccta agattcgtga ttgactcgag tctggt                              756
```

<210> 21
<211> 867
<212> DNA
<213> Unknown

<220>
<223> Séquence nucléotidique de la région pertinente du vecteur permettant l'expression d'une protéine de fusion ayant des dérivés de Jo et In localisées respectivement en N et C-terminus de la boucle 1 du canal potassique

<220>
<221> CDS
<222> (1)..(855)

<220>
<221> misc_feature
<222> (13)..(30)
<223> Séquence nucléotidique codant pour 6 histidines

<220>
<221> misc_feature
<222> (43)..(279)
<223> Séquence nucléotidique codant pour un dérivé de Jo

<220>
<221> misc_feature
<222> (280)..(288)
<223> Séquence nucléotidique codant pour des acides aminés du linker

<220>
<221> misc_feature
<222> (289)..(447)
<223> Séquence nucléotidique codant pour la boucle 1 du canal potassique

<220>
<221> misc_feature
<222> (448)..(453)
<223> Séquence nucléotidique codant pour des acides aminés du linker

<220>
<221> misc_feature
<222> (454)..(855)
<223> Séquence nucléotidique codant pour un dérivé de In

<400> 21

<220>
<221> misc_feature
<222> (448)..(453)

```
atg ggc agc agc cat cac cat cat cac cac agc cag gat ccg tct gac         48
Met Gly Ser Ser His His His His His His Ser Gln Asp Pro Ser Asp
1               5                   10              15

cag tat cca caa aca ggg act tat cca gat gtt caa aca cct tat cag         96
Gln Tyr Pro Gln Thr Gly Thr Tyr Pro Asp Val Gln Thr Pro Tyr Gln
            20                  25                  30

att att aag gta gat ggt tcg gaa aaa aac gga cag cac aag gcg ttg        144
Ile Ile Lys Val Asp Gly Ser Glu Lys Asn Gly Gln His Lys Ala Leu
        35                  40                  45

aat ccg aat cca tat gaa cgt gtg att cca gaa ggt aca ctt tca aag        192
Asn Pro Asn Pro Tyr Glu Arg Val Ile Pro Glu Gly Thr Leu Ser Lys
    50                  55                  60

aga att tat caa gtg aat aat ttg gat gat aac caa tat gga atc gaa        240
Arg Ile Tyr Gln Val Asn Asn Leu Asp Asp Asn Gln Tyr Gly Ile Glu
65                  70                  75                  80

ttg acg gtt agt ggg aaa aca gtg tat gaa caa aaa gat gga att cca        288
Leu Thr Val Ser Gly Lys Thr Val Tyr Glu Gln Lys Asp Gly Ile Pro
                85                  90                  95

gaa att tct cag cgt acg acc att gtg att cag aaa caa acg ttt atc        336
Glu Ile Ser Gln Arg Thr Thr Ile Val Ile Gln Lys Gln Thr Phe Ile
            100                 105                 110

agt cag cat tcc tgt gtg aat agc acc gaa ctg gat gaa ctg atc caa        384
Ser Gln His Ser Cys Val Asn Ser Thr Glu Leu Asp Glu Leu Ile Gln
            115                 120                 125

caa att gtg gca gcg atc aat gcc ggt att att cca ctg ggg aac aca        432
Gln Ile Val Ala Ala Ile Asn Ala Gly Ile Ile Pro Leu Gly Asn Thr
        130                 135                 140

tca aac caa atc agc aag ctt act gaa aag aaa tca att gag aat ggt        480
Ser Asn Gln Ile Ser Lys Leu Thr Glu Lys Lys Ser Ile Glu Asn Gly
145                 150                 155                 160

acg att aca gat ccg atg ggt gag tta att gat ttg caa ttg ggc aca        528
Thr Ile Thr Asp Pro Met Gly Glu Leu Ile Asp Leu Gln Leu Gly Thr
                165                 170                 175

gat gga aga ttt gat cca gca gat tac act tta act gca aac gat ggt        576
Asp Gly Arg Phe Asp Pro Ala Asp Tyr Thr Leu Thr Ala Asn Asp Gly
            180                 185                 190

agt cgc ttg gag aat gga caa gct gta ggt ggt cca caa aat gat ggt        624
Ser Arg Leu Glu Asn Gly Gln Ala Val Gly Gly Pro Gln Asn Asp Gly
        195                 200                 205

ggt ttg tta aaa aat gca aaa gtg ctc tat gat acg act gag aaa agg        672
Gly Leu Leu Lys Asn Ala Lys Val Leu Tyr Asp Thr Thr Glu Lys Arg
    210                 215                 220

att cgt gta aca ggt ctg tac ctt gga acg gat gaa aaa gtt acg ttg        720
Ile Arg Val Thr Gly Leu Tyr Leu Gly Thr Asp Glu Lys Val Thr Leu
```

```
      225                        230                        235                        240

      acc tac aat gtt cgt ttg aat gat gag ttt gta agc aat aaa ttt tat        768
      Thr Tyr Asn Val Arg Leu Asn Asp Glu Phe Val Ser Asn Lys Phe Tyr
                      245                        250                        255

      gat acc aat ggt cga aca acc tta cat cct aag gaa gta gaa cag aac        816
      Asp Thr Asn Gly Arg Thr Thr Leu His Pro Lys Glu Val Glu Gln Asn
                      260                        265                        270

      aca gtg cgc gac ttc ccg att cct aag att cgt gat tga ctcgagtctg gt      867
      Thr Val Arg Asp Phe Pro Ile Pro Lys Ile Arg Asp
                      275                        280
```

<210> 22
<211> 284
<212> PRT
<213> Unknown

<220>
<223> Synthetic Construct

<400> 22

```
Met Gly Ser Ser His His His His His His Ser Gln Asp Pro Ser Asp
1                5                    10                  15

Gln Tyr Pro Gln Thr Gly Thr Tyr Pro Asp Val Gln Thr Pro Tyr Gln
            20                  25                  30

Ile Ile Lys Val Asp Gly Ser Glu Lys Asn Gly Gln His Lys Ala Leu
            35                  40                  45

Asn Pro Asn Pro Tyr Glu Arg Val Ile Pro Glu Gly Thr Leu Ser Lys
    50                  55                  60

Arg Ile Tyr Gln Val Asn Asn Leu Asp Asp Asn Gln Tyr Gly Ile Glu
65                  70                  75                  80

Leu Thr Val Ser Gly Lys Thr Val Tyr Glu Gln Lys Asp Gly Ile Pro
                85                  90                  95

Glu Ile Ser Gln Arg Thr Thr Ile Val Ile Gln Lys Gln Thr Phe Ile
            100                 105                 110

Ser Gln His Ser Cys Val Asn Ser Thr Glu Leu Asp Glu Leu Ile Gln
            115                 120                 125

Gln Ile Val Ala Ala Ile Asn Ala Gly Ile Ile Pro Leu Gly Asn Thr
    130                 135                 140

Ser Asn Gln Ile Ser Lys Leu Thr Glu Lys Lys Ser Ile Glu Asn Gly
145                 150                 155                 160
```

```
Thr Ile Thr Asp Pro Met Gly Glu Leu Ile Asp Leu Gln Leu Gly Thr
                165                 170                 175

Asp Gly Arg Phe Asp Pro Ala Asp Tyr Thr Leu Thr Ala Asn Asp Gly
            180                 185                 190

Ser Arg Leu Glu Asn Gly Gln Ala Val Gly Gly Pro Gln Asn Asp Gly
            195                 200                 205

Gly Leu Leu Lys Asn Ala Lys Val Leu Tyr Asp Thr Thr Glu Lys Arg
    210                 215                 220

Ile Arg Val Thr Gly Leu Tyr Leu Gly Thr Asp Glu Lys Val Thr Leu
225                 230                 235                 240

Thr Tyr Asn Val Arg Leu Asn Asp Glu Phe Val Ser Asn Lys Phe Tyr
                245                 250                 255

Asp Thr Asn Gly Arg Thr Thr Leu His Pro Lys Glu Val Glu Gln Asn
            260                 265                 270

Thr Val Arg Asp Phe Pro Ile Pro Lys Ile Arg Asp
    275                 280
```